(19) Europäisches Patentamt
European Patent Office
Office européen des brevets

(11) Publication number: **0 296 580 B1**

(12) **EUROPEAN PATENT SPECIFICATION**

(45) Date of publication of patent specification: **11.11.92**

(51) Int. Cl.5: **C07C 69/732**, C07C 59/48, C07D 307/54, C07D 333/24, A61K 31/215, A61K 31/34, A61K 31/38, C07D 309/10

(21) Application number: **88109997.2**

(22) Date of filing: **23.06.88**

(54) **Phenylene, furyl, and thienyl leukotriene B4 analogues.**

(30) Priority: **26.06.87 US 67526**
**03.06.88 US 202279**

(43) Date of publication of application:
**28.12.88 Bulletin 88/52**

(45) Publication of the grant of the patent:
**11.11.92 Bulletin 92/46**

(84) Designated Contracting States:
**AT BE CH DE ES FR GB GR IT LI NL SE**

(56) References cited:

**JOURNAL OF ORGANIC METALLIC CHEMISTRY, vol. 311, no. 3, 9th September 1986, pages C35-C38, Elsevier Sequoia S.A., Lausanne, CH; M. FURBER et al.: "An improved procedure for the generation and selective trapping of 2,4'-dilithiophenylethyne"**

**THE JOURNAL OF THE CHEMICAL SOCIETY PERKIN TRANSACTIONS I, no. 7, July 1987, pages 1573-1578, Royal Society of Chemistry, London, GB; M. FURBER et al.: "The synthesis of aromatic leukotriene analogues; regioselective trapping of 2,4'-dilithiophenylethyne"**

(73) Proprietor: **G.D. Searle & Co.**
**P.O. Box 5110**
**Chicago Illinois 60680(US)**

(72) Inventor: **Djuric, Stevan Wakefield**
**1023 Huber Lane**
**Glenview, IL 60025(US)**
Inventor: **Haack, Richard Arthur**
**5431 Christiana Avenue**
**Chicago, IL 60625(US)**
Inventor: **Miyashiro, Julie Marion**
**746 Hinman**
**Evanston, IL 60202(US)**

(74) Representative: **Wolff, Hans Joachim, Dr.jur. Dipl.-Chem. et al**
**Beil, Wolff & Beil Rechtsanwälte Postfach 80 01 40 Adelonstrasse 58**
**W-6230 Frankfurt am Main 80(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

(a) Field of the Invention

The present invention relates to pharmaceutical agents (compounds) which act as leukotriene $B_4$ - ($LTB_4$) antagonists in mammals. The compounds of the present invention are useful in treating inflammatory conditions in mammals such as psoriasis, Crohn's disease, ulcerative colitis and the like.

(b) Prior Art

$LTB_4$ (Formula I) is an arachidonic acid metabolite which is an important mediator of inflammation in mammals. As a mediator of inflammation $LTB_4$ is known to induce chemotaxis, chemokinesis aggregation, and degranulation of leukocytes in vitro, and to induce accumulation of polymorphonuclear leukocytes, and increase vascular permeability and edema formation in vivo.

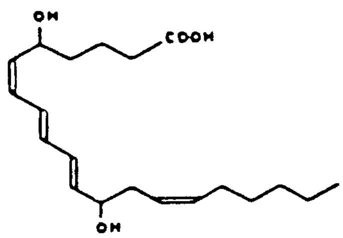

I

Particularly high levels of $LTB_4$ are detected in lesions in inflammatory diseases such as rheumatoid or spondylarthritis, gout, psoriasis, ulcerative colitis, Crohn's disease, and some respiratory diseases.

Accordingly, it is an object of this invention to produce compounds for use as pharmaceutical agents which will exhibit $LTB_4$ antagonist activity in mammals.

A potential $LTB_4$ antagonist (Formula II), which is structurally different from the compounds of the present invention, is disclosed in Biochem. and Biophys. Res. Comm., 138 540-546 (1986).

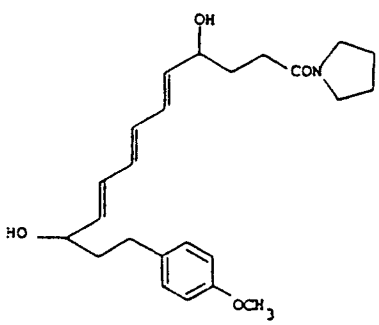

II

In this article, the authors also suggest that they have found antagonistic activity in a series of unidentified unsaturated dihydroxy fatty acid derivatives which are to be the subject of a future publication.

The pharmacology of the biologically active leukotrienes is generally discussed in J. Clin. Invest. 73, 889.897 (1984).

The J.Chem.Soc.Perk.Trans. I, 1987, published July, 1987 after the 1st and before the 2nd priority of the present application discloses a method for preparing $LTB_4$-antagonists with a basic phenyl or pyridyl group. These are structurally different from the presently claimed relevant compounds having a basic thienyl group belonging to the present 2nd priority compounds. Said document does not give any hint how to modify known $LTB_4$-antagonists to obtain highly useful antiinflammatory agents.

The J. Org. Med. Chem., 1986, pages C35-C38 describes an improved procedure for the generation and selective trapping of 2,4'-dilithiophenylethyne, among others the preparation of methyl-7-[4-(hydroxynonyl)phenyl]-5-hydroxy-6-heptynoate. The reference, however, is silent about any pharmaceutical efficacy of the said compound.

## SUMMARY OF THE INVENTION

This invention encompasses compounds of the formula

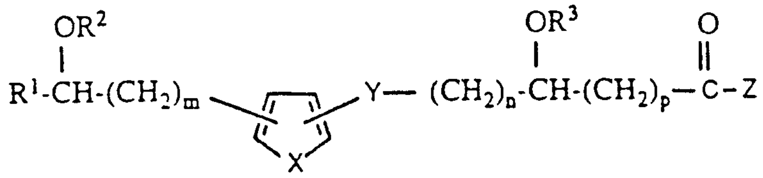

III

and the pharmaceutically acceptable non-toxic addition salts thereof;

wherein $R^1$ is lower alkyl having 1-10 carbon atoms; lower alkenyl having 2-10 carbon atoms; lower alkynyl and having 2-10 carbon atoms; lower alkadienyl having 3-10 carbon atoms; lower alkadiynyl having 4-10 carbon atoms; or alkenynyl having 4-10 carbon atoms;

wherein $R^2$ and $R^3$ are the same or different and represent hydrogen or lower alkyl having 1-6 carbon atoms;

wherein X is CH = CH, S, or O;

wherein Y is CH = CH or C≡C;

wherein Z is $OR^4$ or $NR^5R^6$, and wherein $R^4$ represents H, lower alkyl having 1-6 carbon atoms, or a pharmaceutically acceptable cation, and wherein $R^5$ and $R^6$ act independently and represent H or lower alkyl having 1-6 carbon atoms, or $R^5$ and $R^6$ may act together with N to form a cycloamine of the formula:

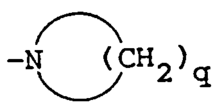

IV

wherein q is an integer from 2-5;

wherein m and n are the same or different and either 1 or 0; and wherein p is an integer from 1 to 5.

The invention does not encompass the known compound methyl-7-[4-(hydroxynonyl)phenyl]-5-hydroxy-6-heptynoate, but is directed to the use of said compound as $LTB_4$-antagonist.

## DETAILED DESCRIPTION

This invention encompasses compounds of Formula III as previously described including any stereoisomers thereof. A particularly preferred embodiment of the present invention is encompassed by a compound of the formula:

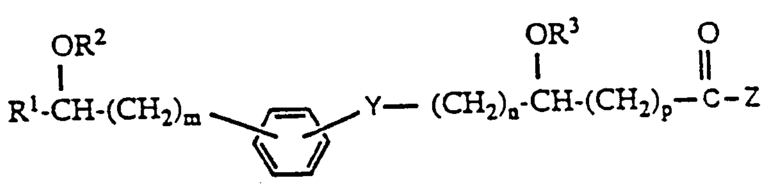

V

wherein $R^1$, $R^2$, $R^3$, Y, Z, $R^4$, $R^5$, $R^6$, m, n, p, and q are as previously defined for Formulas III and IV and with the exception as indicated.

The term "alkyl" as used to described $R^1$, $R^2$, $R^3$, $R^4$, $R^5$, and $R^6$ means straight or branched chain alkyls having 1-10 carbon atoms.

The term "alkenyl" as used to describe $R^1$ means straight or branched chain alkenyls having 2-10 carbon atoms.

The term "alkynyl" as used to describe $R^1$ means straight or branched chain alkynyls having 2-10 carbon atoms.

The term "alkadienyl" as used to describe $R^1$ means straight or branched chain alkadienes, including allenes, having 3-10 carbon atoms.

The term "alkadiynyl" as used to describe $R^1$ means straight or branched chain alkadiynyls having 4-10 carbon atoms.

The term "alkenynyl" as used to describe $R^1$ means straight or branched chain alkenynyls having 4-10 carbon atoms.

The term "pharmaceutically acceptable cations" as used to describe $R^4$ refers to cations such as ammonium, sodium, potassium, lithium, calcium, magnesium, ferrous, zinc, copper, manganous, aluminum, ferric manganic, ammonium, tetraalkyl-ammonium and the like.

The term "pharmaceutically acceptable non-toxic addition salts" refers either to those base derived salts of any compound herein having a carboxylic acid function.

The base derived salts may be derived from pharmaceutically acceptable non-toxic inorganic or organic bases. Among the inorganic bases employed to produce said pharmaceutically acceptable salts are the hydroxide bases of the "pharmaceutically acceptable cations" disclosed above.

Among the organic bases employed to produce said pharmaceutically acceptable salts are the pharmaceutically acceptable non-toxic bases of primary, secondary, and tertiary amines. Especially preferred non-toxic bases are isopropylamine, diethylamine, ethanolamine, dicyclohexylamine, choline, and caffeine.

All the pharmaceutically acceptable non-toxic addition salts are prepared by conventional processes well known to those of ordinary skill in the art.

The compounds of this invention are generally prepared by separately adding two chains to an appropriately substituted aromatic moiety. The first chain can be added by initially performing a nucleophilic addition of a bromoalk-1-yne compound, such as via a Grignard reaction to a bromo-substituted aromatic aldehyde. The aromatic moiety can be phenyl, thienyl or furyl. The Grignard reagent adds to the aldehyde group to form an alkynol compound. The resulting hydroxyl group is typically protected by reaction with a trialkylchlorosilane, preferably t-butyldimethylchlorosilane.

The length of the alkyne side chain can be optionally increased to produce an $R^1$ of the desired length. One method of increasing the chain length is to convert the terminal acetylene into an anion by reaction with an alkyl lithium compound in an aprotic solvent. This anion can then be added to a straight or branched chain alkyl iodide via a nucleophilic substitution. By varying the chain lengths of the bromoalkyne and the iodide compound in the above reaction, the necessary variations can be achieved to produce the $R^1$ substituents claimed in this invention.

The second chain can be added to the above aromatic moiety via a catalytic reaction. By selecting a hydroxyester containing a terminal triple bond and by protecting the hydroxyl group with a trialkylsilane, preferably t-butyldimethylchlorosilane, one can substitute the terminal acetylene for the bromo on the aromatic moiety. By varying the chain length and the position of the hydroxyl group, one can achieve the necessary variations to produce diyne compounds encompassed by the present invention.

The diyne compounds can be catalytically hydrogenated over Lindlar catalyst to produce diene compounds also encompassed by the present invention.

The biological activity possessed by the compounds of this invention was indicated by positive results to the "$LTB_4$ Receptor Binding Assay" and the "Human Neutrophil Degranulation Assay".

Preparation of Human Neutrophils:

For use in both the "$LTB_4$ receptor Binding Assay" and the "human Neutrophil Degranulation Assay", neutrophils were purified from venous blood of normal human donors using standard techniques of dextran sedimentation, centrifugation on Histopaque® (density solution) and hypotonic lysis of erythrocytes (Boyum, A., Isolation of Leukocytes From Human Blood: Further Observations. Scand. J. Lab. Clin. Inves. 21 (Suppl. 97): 31, 1968). The purity of isolated neutrophils was ≧ 95%.

$LTB_4$ Receptor Binding Assay:

Neutrophils (4-6x$10^6$) in 1ml of Hanks' balanced salt solution containing 10mM Hepes Buffer (HBSS), pH 7.4 and 30 $\mu$M nordihydroguaiaretic acid were incubated with 0.6nM ($^3$H) $LTB_4$ in the presence or absence of test compounds. The incubation was carried out at 0°C for 45 minutes and terminated by adding 5ml of ice-cold HBSS followed by rapid filtration of incubation mixture under vacuum through GF/C glass fiber filters. The filters were further washed with 10ml HBSS and their radioactivity was determined. Specific binding was defined as the difference between total binding and nonspecific binding which was not displaced by $10^{-7}$M unlabeled $LTB_4$.

The inhibition of specific binding was determined for representatives compounds of this invention, and the corresponding $IC_{50}$ values calculated (Table 1). An $IC_{50}$ is the concentration of the compound of interest which will inhibit the binding of $LTB_4$ by 50% of the $LTB_4$ receptors. For example, for the compound of Example 7, the $IC_{50}$ was determined to be approximately 5$\mu$M.

Human Neutrophil Degranulation Assay:

$LTB_4$ induced neutrophil degranulation was determined by measuring the release of myeloperoxidase activity into the incubation medium. Neutrophils (3 x $10^6$) in 1ml HBSS solution were preincubated with cytochalasin B(5$\mu$g) at 37°C for 5 minutes, followed by preincubation with test compounds for 7 minutes. Neutrophils were then incubated for 2 to 20 minutes with either $LTB_4$(5 x $10^{-8}$M) or the chemotactic peptide f-met-leu-phe (5 x $10^{-6}$M) to induce degranulation. Following incubation, samples were centrifuged and myleoperoxidase was extracted from the cell pellets by sonication in phosphate buffer containing 0.4% Triton X-100. Triton X-100 was also added to the supernatents to a concentration of 0.4%. The supernatants and the pellet extracts were then assayed spectrophotometrically for myeloperoxide activity by determining the rate of decomposition of $H_2O_2$ with o-dianisidine as hydrogen donor as described by Renlund D.G., MacFarlane J.L., Christensen, R.D., Lynch R.E., and Rothstein, G., A Quantitative And Sensitive Method For Measurement Of Myeloperoxidase, Clinical Research 28:75A, 1980). Myeloperoxidase activity released into the supernatant was expressed as the percent of the average total activity (pellet plus supernatant).

The inhibition of $LTB_4$ induced neutrophil degranulation was determined for representative compounds of this invention and their corresponding $IC_{50}$ values were calculated (Table 1). The concentration of a compound which inhibited $LTB_4$ induced neutrophil degranulation by 50% was determined to be its $IC_{50}$ value.

By virtue of their activity as $LTB_4$ antagonists, the compounds of Formula I are useful in treating inflammatory conditions in mammals such as psoriasis, Crohn's disease, ulcerative colitis and the like. A physician or veterinarian of ordinary skill can readily determine whether a subject exhibits the inflammatory condition. The preferred utility relates to treatment of ulcerative colitis.

The compounds of the present invention can be administered in such oral dosage forms as tablets, capsules, softgels, pills, powders, granules, elixirs, or syrups. The compounds may also be administered intravascularly, intraperitoneally, subcutaneously, intramuscularly, or topically using forms known to the pharmaceutical art. In general, the preferred form of administration is oral. For the orally administered pharmaceutical compositions and methods of the present invention, the foregoing active ingredients will typically be administered in admixture with suitable pharmaceutical diluents, excipients, or carriers (collectively referred to herein as "carrier" materials) suitably selected with respect to the intended form of administration, that is, oral tablets, capsules, softgels, elixirs, syrups, drops, and the like, and consistent with conventional pharmaceutical practices.

For example, for oral administration in the form of tablets or capsules, a therapeutically effective amount of one or more compounds of the present invention may be combined with any oral non-toxic pharmaceutically acceptable inert carrier such as lactose, starch, sucrose, cellulose, magnesium stearate, dicalcium phosphate, calcium sulfate, mannitol, and the like, or various combinations thereof. For oral administration in liquid forms, such as in softgels, elixirs, syrups, drops and the like, a therapeutically effective amount of the active drug components may be combined with any oral non-toxic pharmaceutically acceptable inert carrier such as water, saline, ethanol, polyethylene glycol, propylene glycol, corn oil, cottonseed oil, peanut oil, sesame oil, benzyl alcohol, various buffers, and the like, or various combinations thereof. Moreover, when desired or necessary, suitable binders, lubricants, disintegrating agents, and coloring agents can also be incorporated in the mixture. Suitable binders include starch, gelatin, natural sugars, corn sweeteners, natural and synthetic gums such as acacia, sodium alginate, carboxymethylcellulose, polyethylene glycol, and waxes or combinations thereof. Lubricants for use in these dosage forms include boric acid, sodium benzoate, sodium acetate, sodium chloride, and the like or combinations thereof. Disintegrators include, without limitation, starch, methylcellulose, agar, bentonite, guar gum, and the like, or combinations thereof. Sweetening and flavoring agents and preservatives can also be included where appropriate.

For intravascular, intraperitoneal, subcutaneous, or intramuscular administration, one or more compounds of the present invention may be combined with a suitable carrier such as water, saline, aqueous dextrose, and the like. For topical administration, such as for psoriasis, therapeutically effective amounts of one or more compounds of the present invention can be combined with pharmaceutically acceptable creams, oils, waxes, gels and the like. Regardless of the route of administration selected, the compounds of the present invention are formulated into pharmaceutically acceptable dosage forms by conventional methods known to those skilled in the art. The compounds may also be formulated using pharmacologically acceptable base addition salts. Moreover the compounds or their salts may be used in a suitable hydrated form.

Regardless of the route of administration selected, a non-toxic but therapeutically effective quantity of one or more compounds of this invention is employed in any treatment. The dosage regimen for preventing or treating inflammatory conditions with the compounds of this invention is selected in accordance with a variety of factors, including the type, age, weight, sex, and medical condition of the patient, the severity of the inflammatory condition, the route of administration, and the particular compound employed in the treatment. A physician or veterinarian of ordinary skill can readily determine and prescribe the effective amount of the drug required to prevent or arrest the progress of the condition. In so proceeding, the physician or veterinarian could employe relatively low doses at first and subsequently increase the dose until a maximum response is obtained. Daily dosages of the compounds of the invention are ordinarily in the range of about 1.0 mg/kg up to about 21.0 mg/kg, [preferably in the range of about 2.0 to 14.0 mg/kg (orally)].

The following examples illustrate the methods used to prepare the compounds of this invention.

In the following examples, and throughout this application a wavey line ( ) defines a substituent as having optional R or S stereochemistry. A broken triangular shaped line ( ) defines the substituent at the base of the triangle as coming out of the plane of the paper, whereas a substituent at the apex of the broken triangle, is defined as going into the plane of the paper.

Table 1 Biological Activity For Representative Compounds Of The Invention

| Compound (Example No.) | Structure | Inhibition of Receptor Binding of $LTB_4$ $IC_{50}$( M) | Inhibition of $LTB_4$ Induced Neutrophil Degranulation $IC_{50}$( M) |
|---|---|---|---|
| 7 | 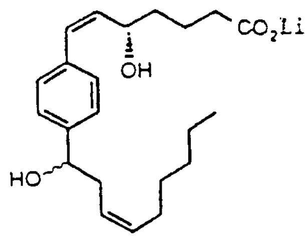 | 5 | 0.7 |
| 11 | 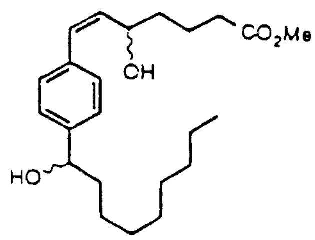 | 1 | 0.65 |
| 21 | 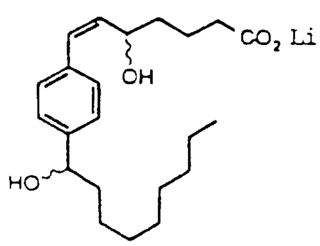 | 2 | 1.0 |

| | | | |
|---|---|---|---|
| 23 | 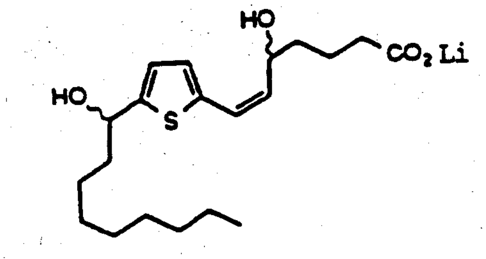 | 5 | 1.8 |
| 22 | 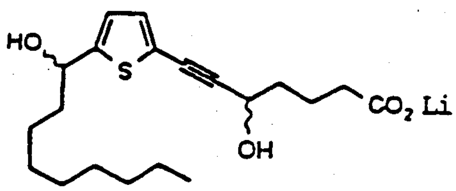 | 20% inhibition at 10μM | 8.7 |

DESCRIPTION OF THE PREFERRED EMBODIMENT

Example 1

1-(p-bromophenyl)-but-3-yn-1-ol

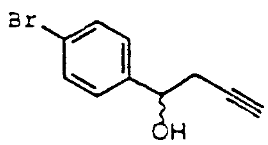

To 4.1g of flame dried Mg was added 50ml of diethyl ether ("ether") followed by the addition of a few iodine crystals. To this was added a 10ml aliquot of a solution containing 15ml (168.9 mmol) of propargyl bromide in 50ml of ether. The reaction was started by the addition of 25mg of $HgCl_2$. The remaining solution of propargyl bromide in ether was then added at a rate sufficient to maintain a steady reflux. Once addition was complete, the mixture was stirred 1 hour at room temperature (R.T.) and then placed in an ice bath and cooled to 0 ° C. To the cooled reaction mixture was added dropwise with stirring over a 1 hour period, a solution containing 25g (135.1 mmol) of 4-bromobenzaldehyde dissolved in 30ml of ether and 30ml of THF. Once addition was complete, the ice bath was removed and the reaction mixture stirred overnight at room temperature (R.T.). The reaction was quenched with a saturated $NH_4Cl$ solution. The layers were separated and the aqueous layer was extracted twice with ether. The combined extracts were washed 1X each with $H_2O$, and brine and then dried ($MgSO_4$). Removal of the solvent produced 30.2g of a crude yellow oil which was semi-purified by high pressure liquid chromatography, HPLC, (silica; gradient elution with methyl t-butyl ether-hexane) to yield 20.8g of a reaction mixture that was 80% pure in the titled product.

Example 2

1-(p-bromophenyl)-1-(t-butyldimethylsiloxy)-3-butyne

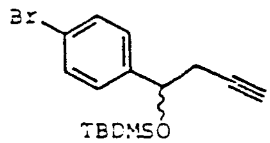

TBDMSO = t-butyldimethylsiloxy

A solution containing 19g (84.5 mmol) of the semi-purified reaction product of Example 1 dissolved in 50ml of DMF was cooled to 0 ° C (ice bath) and 12.9g (190 mmol) of imidazole was added in one portion. The reaction mixture was stirred 10 minutes until all the imidazole dissolved and then was added in one portion 14.3g (95 mmol) of t-butyldimethylchlorosilane. After stirring for 5 minutes, the ice bath was removed and the reaction mixture was stirred for an additional 2 hr. at R.T. The reaction was then poured into 500ml of ether and washed 3x with 50cc of $H_2O$ and 1x with 100ml of brine. The organic layer was then separated, dried ($MgSO_4$), and removal of all solvent yielded 29.32g of crude product. Separation by reverse phase HPLC (gradient elution with acetonitrile-water) yielded 9.1g of the titled product.

| Analysis for $C_{16}H_{23}OSiBr$ (MW = 339.35): | | | |
|---|---|---|---|
| Calcd:<br>Found: | C, 56.63;<br>C, 56.61; | H, 6.83;<br>H, 6.88; | Br, 23.55.<br>Br, 23.56. |

Example 3

1-(p-bromophenyl)-1-(t-butyldimethylsiloxy)-3-nonyne

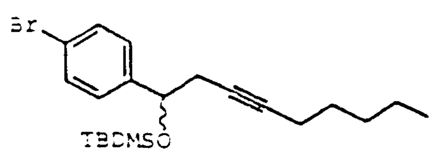

TBDMSO = t-butyldimethylsiloxy

To 2.2g (63 mmol) of the silylacetylene of Example 2 dissolved in 50ml of dry THF and cooled to -78 ° C under argon was added dropwise 5.6ml (7.0 mmol) of a 1.25M solution of methyl lithium in diethyl ether. Upon addition, the reaction was warmed to -10 ° C and stirred at -10 ° C for 30 min. Iodopentane (1.2ml, 9.0 mmol) was then added followed by 5.0ml of hexamethylphosphoric triamide (HMPA) whereupon the reaction mixture was warmed to R.T. and stirred overnight. The reaction was quenched with about 5.0ml of $H_2O$, then poured into hexane and washed 4x with $H_2O$, and 1x with brine. The organic layer was dried ($MgSO_4$) and the solvent removed to yield a dark red oil which was purified by medium pressure liquid chromotography (MPLC) eluting with hexane to yield 2.14g of the titled product as a pale yellow oil.

1H N.M.R $\delta_{TMS}$ $CDCl_3$ (300 MHz):
-0.08(s,3H); 0.03(s,3H); 0.9(s&t, 12H); 1.3(br.m,4H); 1.43(br.m,2H); 2.1(tt,2H); 2.45(m,2H); 4.72(t,1H); 7.32-(dd,4H).

Example 4

Methyl 7-[4-[1-(t-butyldimethylsiloxy)-3-nonynyl]phenyl]-5S-(t-butyldimethylsiloxy)-6-heptynoate

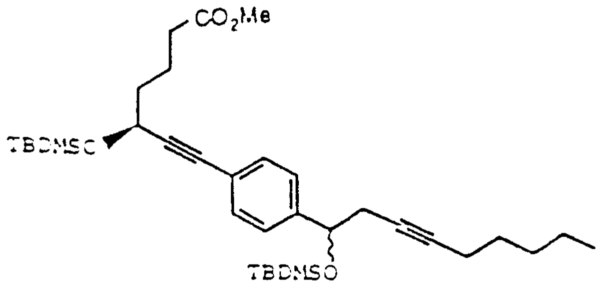

The following reagents were added to a pressure vessel: 0.1g (0.24 mmol) of 2-(t-butyldimethylsiloxy)-1-p-bromophenyl-3-nonyne, 0.065g (0.24 mmol) of methyl 5S-(t-butyldimethylsiloxy)-6-heptynoate prepared according to the procedure of Nicolaou et al., J.A.C.S., 106, 2748 (1984) employing the optically active 5S-alcohol, 1ml of piperidine, and 6mg (.005 mole, 2 mole%) of $Pd(PPh_3)_4$. The vessel was degassed with argon, sealed and the reaction mixture was then heated at 100-120°C for 2 hr. with stirring. The reaction was then cooled to room temperature, diluted with diethyl ether, filtered, and stripped of all solvent. The residue was purified by MPLC, eluting with 2.5% ethyl acetate-hexane to yield 0.7g of the titled product.

$^1$H N.M.R. $\delta_{TMS}$ $CDCl_3$ (300 MHz):
-0.08(s, 3H); 0.04(s, 3H); 0.15(s, 3H); 0.17(s, 3H); 0.88(s&t, 12H); 0.92(s, 9H); 1.29(m, 4H); 1.43(m, 2H); 1.79-(m, 4H); 2.09(tt, 2H); 2.35-2.6(complex, 4H); 3.66(s, 3H); 4.59(t, 1H); 4.74(t, 1H); 7.3(dd, 4H).

## Example 5

Methyl 7-[4-[1-(t-butyldimethylsiloxy)-3Z-nonenyl] phenyl]-5S-(t-butyldimethylsiloxy)-6Z-heptenoate

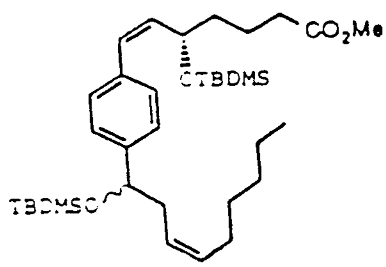

To 0.07g of the titled product of Example 4 in 10ml of hexane was added 0.1ml of quinoline and 10mg of Lindlar catalyst. The mixture was stirred under a $H_2$ atmosphere for 7 hours at room temperature. The reaction was recharged with an additional 10mg of catalyst and the reaction was permitted to run overnight. The reaction mixture was filtered through Celite ® (diatomaceous earth) and the filtrate was evaporated to give an oil. Purification by MPLC eluting with 2.5% ethyl acetate-hexane produced .060g of the titled product.

$^1$H N.M.R. $\delta_{TMS}$ $CDCl_3$ (300 MHz):

-0.15(s, 3H); -0.1(s, 3H); 0.02(s, 3H); 0.05(s, 3H); 0.85(s, 9H); 0.9(s&t, 12H); 1.28(br.m, 4H); 1.55-1.9(br.m, 6H); 1.96(m, 2H); 2.34-2.6(br.m&t, 4H); 3.7(s, 3H); 4.65(m, 2H); 5.40(m, 2H); 5.65(dd 1H); 6.44(d, 1H); 7.2-(dd, 4H).

## Example 6

Mixture of methyl 7-[4-(1-hydroxy-3Z-nonenyl)phenyl]-5S-hydroxy-6Z-heptenoate

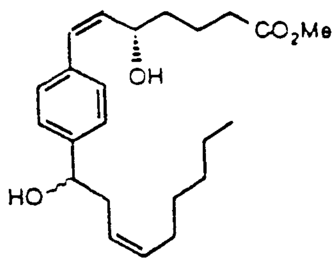

(A)

and

Tetrahydro-6S-[2-[4-(1-hydroxy-3Z-nonenyl)phenyl]-Z-ethenyl]-2H-pyran-2-one

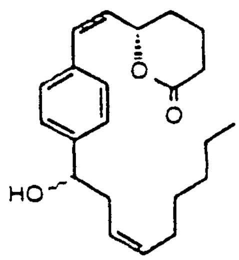

(B)

To 0.32g (0.53 mmol) of the product of Example 5 dissolved in 0.5ml of DMF was added 6.4mg (2.1 eq.) of KF, 1.4mg (0.1 eq.) of 18-crown-6-polyether, and 2µl (2.1 eq.) of $H_2O$. The reaction was stirred under Ar for 24 hr. An additional 6mg of KF was then added and the reaction was stirred overnight at R.T. The reaction mixture was then poured into water and the aqueous solution extracted 3x with ether. The combined extracts were washed 2x with $H_2O$ and 2x with brine and dried ($MgSO_4$). Removal of the solvent in vacuo produced an oil. The oil was purified by flash chromatography (silica, gradient elution with ether - petroleum ether). Fraction I contained .00247g (.0066 mmol) of the ester (A). Fraction II contained .00166g (.0049 mmol) of the lactone (B). Fraction III contained an additional .00609g of the ester and lactone in a 1:4 ratio (determined by $^1H$ N.M.R.)

Lactone:

$^1H$ N.M.R. $\delta_{TMS}$ $CDCl_3$ (300 MHz):

0.88(t, 3H); 1.27(m, 6H); 1.7-2.1(complex, 5H); 2.4-2.7(complex, 4H); 4.63(t, 1H); 5.16(m, 1H); 5.3-5.65(m, 2H); 5.22(dd, 1H); 6.2(d, 1H); 7.33(dd, 4H).

Ester:

$^1H$ N.M.R. $\delta_{TMS}$ $CDCl_3$ (300 MHz):

0.89(t, 3H); 1.25(m, 6H); 1.6-1.8(m, 4H); 2.02(m, 2H); 2.35(t, 2H); 2.4-2.64(m, 4H); 3.68(s, 3H); 4.58(m, 1H); 4.71(t, 1H); 5.4(m, 1H); 5.58(m, 1H); 5.66(dd, 1H); 6.55(d, 1H); 7.3(dd, 4H).

Example 7

7[4-(1-hydroxy-3Z-nonenyl)phenyl]-5S-hydroxy-6Z-heptenoic acid, lithium salt

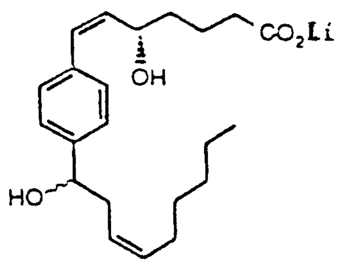

The combined ester and lactone products (0.017 mmol) of Example 6 were dissolved in 0.3ml of methanol and cooled to 0°C. Upon dissolution, 0.1ml of $H_2O$ was added followed by 20µl (.02 mmol) of a 1M LiOH solution. The slurry was stirred for five minutes and then warmed to room temperature. After 24 hrs, an additional 5µl of 1M LiOH was added and stirring continued for an additional 24 hours. The reaction mixture was then evaporated to dryness under a stream of $N_2$ and the last traces of solvent were removed under high vacuum. The reaction produced 6.2mg of the titled product.

$^1H$ N.M.R. $\delta_{d_4Na-TSP}$ $D_2O$ (300 MHz):

5.2-5.5(m, 2H); 5.63(dd, 1H); 6.07(d, 1H); 7.3(dd, 4H).

Example 8

Methyl 7-[4-(1-hydroxy-3-nonynyl)phenyl]-5S-hydroxy-6-heptynoate

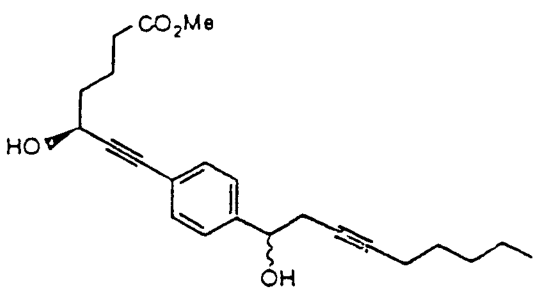

To .044g (0.074 mmol) of the product of Example 4 was added to 0.3ml (0.3 mmol) of 1M tetra-n-butylammonium fluoride in tetrahydrofuran (THF) with stirring at room temperature. The reaction mixture was stirred 6 hours at room temperature and then poured into brine. The brine, containing the reaction mixture, was then extracted 5x with diethyl ether and dried ($MgSO_4$). The dried reaction mixture was then stripped of all solvent and the residue and taken up in 10ml of methanol. To the methanol solution was added 2mg of sodium methoxide and the reaction was stirred overnight. The reaction was stripped in vacuo to give an oil. The oil was purified by flash chromatography (silica; gradient elution with ether-petroleum ether) to give 5mg of the titled product.

$^1H$ N.M.R. $\delta_{TMS}$ $CDCl_3$ (300 MHz):
0.9(t, 3H); 1.3(m, 4H); 1.48(m, 2H); 1.65(broad s, 1H); 1.85(m, 4H); 2.15(m, 3H); 2.42(t, 2H); 2.5-2.65(m, 2H); 3.59(s, 3H); 4.61(broad s, 1H); 4.8(t, 1H); 7.87(dd, 4H).

Example 9

7-[4-(1-hydroxyl-3-nonynyl)phenyl]-5S-hydroxy-6-heptynoic acid, lithium salt

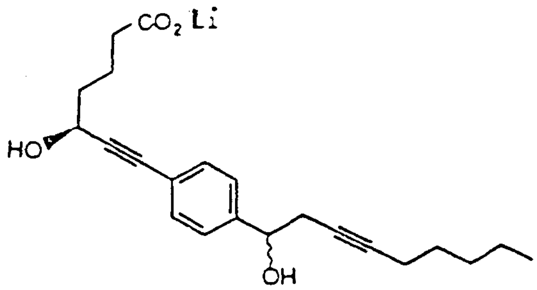

The titled product was prepared according to the reaction described in Example 7, employing 5mg (.0135 mmol) of the product of Example 8 instead of the product of Example 6. The reaction was run until thin layer chromatography (TLC) indicated that all starting material was consumed. The yield was 4.9mg of titled material.

Example 10

1-(p-bromophenyl)-1-nonanol

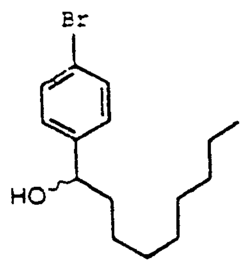

In a 100ml round bottom flask with a Y-adapter, condensor, and a 10ml addition funnel was added 0.38g (15.63 mmol) of Mg turnings which had been ground with a mortar and pestle. The apparatus was then flushed with argon and flame dried. After allowing the apparatus to cool under argon, 4ml of anhydrous diethyl either was added to the Mg. While vigorously stirring the Mg in the ether, 2.52g (13.05 mmol) of 1-bromooctane in 3ml of diethyl ether was added dropwise. After 5 drops, the Grignard reaction started and the addition was continued at a rate sufficient to maintain a steady reflux. After addition was complete, 4ml of dry THF was added to the Grignard reagent and it was stirred at R.T. for an additional 1/2 hour. A warm water bath was then placed under the flask, and refluxing was continued for 15 minutes. Afterwards, the reaction was cooled to 0°C with an ice bath and 2.00g (16.81 mmol) of p-bromobenzaldehyde in 4ml of the THF was added. The reaction was stirred at R.T. for 1.5 hr., whereupon the Grignard was quenched with saturated $NH_4Cl$. The organic layer was washed with $H_2O$ and brine, then dried ($MgSO_4$). Rotary evaporation of the solvent under reduced pressure produced 2.96g of a crude yellow oil. The oil was dissolved in a diethyl ether and loaded onto a silica gel column. Elution with diethyl ether/hexane yielded 2.22g of the titled product as a yellow oil.

| Analysis for $C_{15}H_{23}OBr$ (MW = 299.26): | | |
|---|---|---|
| Calcd: | C, 60.20; | H, 7.75. |
| Found: | C, 60.46; | H, 7.87. |

Example 11

Methyl 7-[4-(hydroxynonyl)phenyl]-5-hydroxy-6-heptynoate

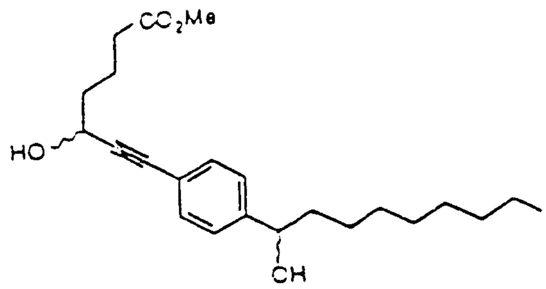

Into a heavy walled Pyrex® tube was added 102mg (0.34 mmol) of 1-(p-bromophenyl)-1-nonanol, 53mg (0.34 mmol) of methyl 5-hydroxy-6-heptynoate prepared according to the procedure of Nicolaou et al., J.A.C.S., 106, 2748 (1984), 10mg (.009 mmol, 2.5 mole%) of $Pd(PPh_3)_4$, and 2ml (1.4g) of diisopropylamine. The tube was degassed with argon, sealed and placed in a hot oil bath at approximately 100 ° C. After about 40 min., a white solid fell out of solution. The reaction was monitored by TLC over the next four hours - some halide was still present, but the acetylene disappeared. The reaction was allowed to cool to R.T. and 40ml of ether was added. The ether solution was extracted with $H_2O$ and brine and then dried ($MgSO_4$). The solvent was stripped from the dried organic phase leaving 100mg of a yellow oil. The oil was taken up in either and chromatographed on a silica gel column eluting with 30% diethyl ether/hexane to produce 40mg of the titled product as a yellow oil

$^1H$ N.M.R. $\delta_{TMS}$ $CDCl_3$ (300 MHz):

7.40(d, 2H); 7.28(d, 2H); 4.57-4.73(broad m, 2H); 3.69(s, 3H); 2.43(broad t, 2H); 2.12(broad d, 1H); 1.10-1.95-(broad m, 19H); 0.88(broad t, 3H).

Example 12

Methyl 7-[4-(1-hydroxynonyl)phenyl]-5-hydroxy-6Z-heptenoate

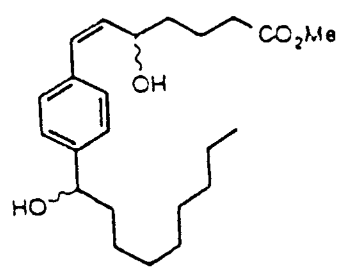

To 2ml of hexane in a 10ml round bottom flask was added 38mg of the titled product of Example 11. The mixture was stirred and benzene was added until the oil went into solution. Upon dissolution, 8mg of Lindlar catalyst and 0.1ml of quinoline were added. The flask was evacuated, flushed 5x with $H_2$, and then stirred under a $H_2$ balloon for 3 days. TLC showed that starting material was still present and an additional 8mg of Lindlar catalyst was added. After 24 hr. there was almost no starting material remaining. Ether (3ml) was added to the reaction mixture and it was filtered through Celite® (diatomaceous earth). The reaction mixture was then sequentially washed with $H_2O$ and brine and then dried ($MgSO_4$). Upon removal of the solvent under reduced pressure, the yellow oil residue was chromatographed on a silica gel column slurry

packed in 70% diethyl ether/hexane. The titled product was recovered as an oil.

| Analysis for $C_{23}H_{36}O_4$ (MW = 376.52): | | |
|---|---|---|
| Calcd: | C, 73.36; | H, 9.64. |
| Found: | C, 73.10; | H, 9.80. |

$^1H$ N.M.R. $\delta_{TMS}$ $CDCl_3$ (300 MHz):

7.33(d, 2H); 7.25(d, 2H); 6.55(d, 1H); 5.65(dd, 1H); 4.67(broad t, 1H); 4.58(broad t, 1H); 3.67(s, 3H); 2.35-(broad t, 2H); 1.20-1.90(broad m, 20H); 0.87(broad t, 3H).

Example 13

1-(o-bromophenyl)-1-nonanol

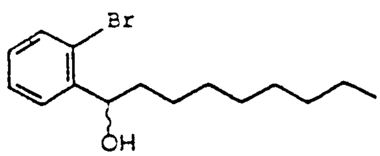

The titled product was prepared according to the procedure of Example 10 substituting o-bromobenzaldehyde for p-bromobenzaldehyde.

Example 14

Methyl 7-[2-(1-hydroxynonyl)phenyl]-5-hydroxy-6-heptynoate

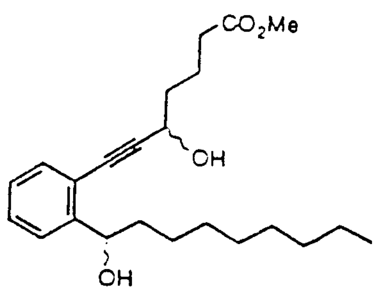

Into a heavy walled Pyrex® test tube was added 130mg (0.43 mmol) of 1-(o-bromophenyl)-1-nonanol, 67mg (0.43 mmol) of methyl 5-hydroxy-6-heptynoate prepared according to the procedure of Nicolaou et al., J.A.C.S., 106, 2748 (1984), 25mg (0.02 mmol, 5 mole%) of $Pd(PPh_3)_4$, and 4ml (93.0mg) of diisopropylamine. The reaction was run and worked up according to the procedure of Example 11 to yield 208mg of a yellow-brown oil. The oil was chromatographed on a silica gel column which was eluted with 70% diethyl ether/hexane to produce the titled product as a yellow oil.

$^1H$ N.M.R. $\delta_{TMS}$ $CDCl_3$ (300 MHz):

7.18-7.55(m, 4H); 5.12(broad t, 1H); 4.65(m, 1H); 3.69(s, 3H); 2.44(broad t, 2H); 1.1-2.30(broad m, 20H);

0.87(broad t, 3H).

Example 15

1-[2-(5-bromo)furanyl]-1-nonanol

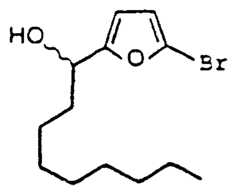

To 0.099g (4.07mmol) of pulverized Mg turnings in 5ml of diethyl ether (in an apparatus set up as in Example 10) was added dropwise with stirring 0.68g (3.52 mmol) of 1-bromooctane in 10ml of diethyl ether. After stirring for 1/2 hour, the reaction mixture was cooled in an ice bath and 20ml of THF was then added. To the reaction mixture was added dropwise with stirring 0.50g (2.86 mmol) of 5-bromo-2-furancarboxaldehyde in 20ml of THF. After the addition was complete, the reaction mixture was stirred for an additional 2 hr. at R.T. The reaction was quenched with saturated $NH_4Cl$ and the organic layer was sequentially extracted with $H_2O$ and brine and then dried ($MgSO_4$). Upon removal of the solvent by rotary evaporation at reduced pressure, 0.79g of a brown oil remained. The oil was chromatographed on silica gel, eluting with 25% ethyl acetate/hexane, to yield 0.50g of the titled product as a yellow oil.

| Analysis for $C_{13}H_{21}O_2Br$ (MW = 289.21): | | |
|---|---|---|
| Calcd: | C, 53.98; | H, 7.32. |
| Found: | C, 54.34; | H, 7.17. |

Example 16

Methyl 7-[2-[5-(1-hydroxynonyl)]furanyl]-5-hydroxy-6-heptynoate

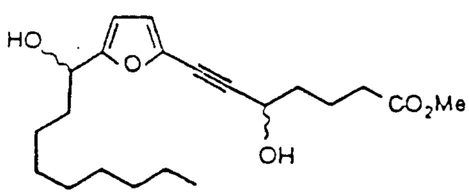

In a heavy walled Pyrex® test tube containing 3ml (2.2g) of diisopropylamine was added 29mg (.10 mmol) of the titled product of Example 15, 16mg (0.10 mmol) of methyl-5-hydroxy-6-heptynoate prepared according to the procedure of Nicolaou et al., J.A.C.S., 106, 1748 (1984), and 9mg (.008mmol, 8 mole%) of $Pd(PPh_3)_4$. The reaction was run and worked up according to the procedure of Example 11 to yield a yellow-brown oil. Chromatography of the oil on a silica gel column eluted with 70% diethyl ether/hexane yielded the titled product as a yellow oil.

$^1H$ N.M.R. $\delta_{TMS}$ $CDCl_3$ (300 MHz):

6.52(d, 1H); 6.22(d, 1H); 4.63(broad m, 2H); 3.68(s, 3H); 2.35-2.45(broad t, 2H); 1.10-2.13(broad m, 20H);

0.88(broad t, 3H).

Example 17

Methyl 7-[2-[5-(1-hydroxynonyl)furanyl]-5-hydroxy-6Z-heptenoate

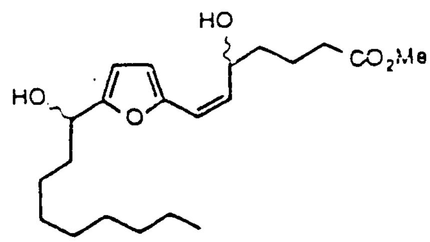

To 16.1mg (0.044 mmol) of the titled product of Example 16, was added 3mg of Lindlar catalyst and 15µl of quinoline. The reaction vessel was then flushed 5x with $H_2$ and the reaction mixture was stirred under a $H_2$ balloon for 1 hour. Afterwards, the reaction mixture was worked up as in Example 12 to product a crude yellow oil. The oil was chromatographed on silica gel column which was eluted with 40% ethyl acetate/hexane to yield 13mg of the titled product as a yellow oil.

$^1H$ N.M.R. $\delta_{TMS}$ $CDCl_3$ (300 MHz):

6.25(s, 2H); 6.15(d, 1H); 5.55(dd, 1H); 5.00(broad t, 1H); 4.65(broad t, 1H); 3.69(s, 3H); 2.40(broad t, 2H); 1.10-1.90(broad m, 20H); 0.87(broad t, 3H).

Example 18

1-[2-(5-bromo)thienyl]-1-nonanol

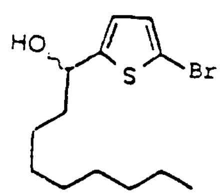

In a 500ml 3-necked round bottom flask equipped with a condensor and a 250ml addition funnel was added 1.66g (68.28 mmol) of Mg. Under a steady flow of argon, the apparatus was flamed. Upon cooling to R.T., 25ml of diethyl ether was added to the Mg turnings, followed by the dropwise addition of 12.21g (63.22 mmol) of 1-bromooctane with vigorous stirring. The Grignard started quickly and addition was continued at a dropwise rate sufficient to maintain steady reflux. After the addition was complete, the reaction was stirred for 1 hr. and 100ml of freshly distilled THF was added to the Grignard which was then cooled in an ice bath. To the cooled Grignard reagent was added 10.00g (52.34 mmol) of 5-bromo-2-thiophenecarboxaldehyde. The reaction mixture was worked up according to the procedure in Example 10. The resulting brown-yellow oil was chromatographed on a silica gel column. Elution with 20% diethyl ether/hexane produced 9.51g of the titled product as a yellow oil.

| Analysis for $C_{13}H_{21}BrOS$ (MW = 305.27): | | | |
|---|---|---|---|
| Calcd: | C, 51.14; | H, 6.93; | Br, 26.18. |
| Found: | C, 51.35: | H, 6.94; | Br, 26.03. |

Example 19

Methyl 7-[2-[5-(1-hydroxynonyl)]thienyl]-5-hydroxy-6-heptynoate

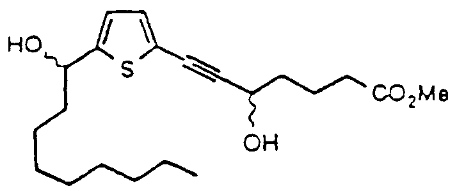

To 30ml of diisopropylamine in a heavy walled Pyrex® test tube was added 283mg (0.93 mmol) of the titled product from Example 18, 143mg (0.92 mmol) of methyl 5-hydroxy-6-heptynoate prepared according to the procedure of Nicolaou et al., J.A.C.S., 106, 2748 (1984), 43mg (0.04 mmol, 4 mole%) of $Pd(PPh_3)_4$. The reaction was run and worked up according to the procedure of Example 11. Chromatography of the resulting oil on silica gel column, which was eluted with 75% diethyl ether/hexane, yielded 66mg of the purified titled product as a yellow oil.

$^1$H N.M.R. $\delta$ $_{TMS}$ $CDCl_3$ (300 MHz):

7.05(d, 1H); 6.80(d, 1H); 4.85(t, 1H); 4.60(broad t, 1H); 3.68(s, 3H); 2.40(broad t, 2H); 2.15-2.30(m, 2H); 1.70-1.90(m, 6H); 1.20-1.40(m, 12H); 0.88(broad t, 3H).

Example 20

Methyl 7-[2-[5-(1-hydroxynonyl)thienyl]-5-hydroxy-6-heptenoate

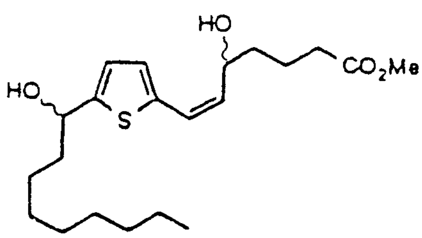

To 42mg of the titled product of Example 19 was added 4mg of 9.5% Lindlar catalyst and 15µl of quinoline. The reaction vessel was flushed 5x with $H_2$ and then stirred under a $H_2$ balloon overnight. An additional 3mg of catalyst was added and the reaction stirred as above for an additional 3 hours. The reaction mixture was diluted 10ml of diethyl ether and filtered through Celite® (diatomaceous earth). The filtrate was washed with $H_2O$ and brine and then dried ($MgSO_4$). Evaporation of the solvent under reduced pressure produced a crude yellow oil. The oil was chromatographed on a silica gel column, which was eluted with 80% diethyl ether/hexane. The resulting titled product was isolated as a yellow oil.

$^1$H N.M.R. $\delta$ $_{TMS}$ $CDCl_3$ (300 MHz):
6.85(broad s, 2H); 6.53(d, 1H); 5.55(dd, 1H); 4.87(m, 2H); 3.68(s, 3H); 2.38(broad t, 2H); 1.17-2.00(broad m,

20H), 0.87(broad t, 3H).

| Analysis for $C_{21}H_{34}O_4S$ (MW = 382.55): | | |
|---|---|---|
| Calcd: | C, 65.93; | H, 8.96. |
| Found: | C, 65.93; | H, 9.15. |

Example 21

7-[4-(1-hydroxynonyl)phenyl]-5-hydroxy-6Z-heptenoic acid, lithium salt

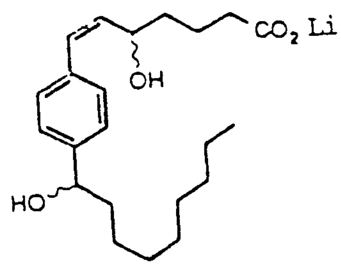

The titled product was prepared according to the reaction described in Example 7 employing the product of Example 12 instead of the product of Example 6. The reaction was run until TLC indicated that all the starting material was consumed.

Example 22

7-[2-[5-(1-hydroxynonyl)thienyl]-5-hydroxy-6Z-heptynoic acid, lithium salt

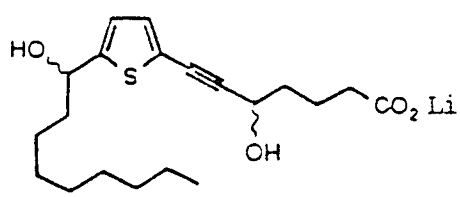

The titled product was prepared according to the reaction described in Example 7 employing the product of Example 19 instead of the product of Example 6. The reaction was run until TLC indicated that all the starting material was consumed.

Example 23

7-[2-[5-(1-hydroxynonyl)thienyl]-5-hydroxy-6Z-heptenoic acid, lithium salt

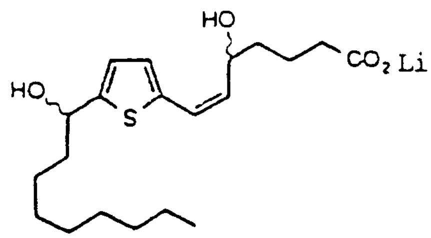

The titled product was prepared according to the reaction described in Example 7 employing the product of Example 20 instead of the product of Example 6. The reaction was run until TLC indicated that all the starting material was consumed.

Example 24

[[1-(5-bromo-2-thienyl)-3-nonyl]oxy](1,1-dimethylethyl)dimethylsilane

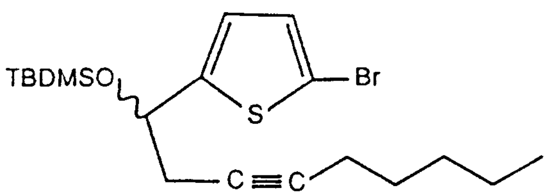

(a) 3-Bromopropyne (48.1g, 404.3 mmol) in 50ml of anhydrous ether was added dropwise to Mg turnings (10.8g, 441.3 mmol) with vigorous stirring. After addition was complete, a few crystals of $HgCl_2$ were added to start the Grignard reaction. The reaction mixture was stirred for 45 min., then cooled in an ice bath and 200 ml of diethyl ether was added. 5-Bromo-2-thiophenecarboxaldehyde (67.4g, 352.5 mmole) in 75ml of dry THF was added dropwise at 0 °C. The reaction mixture was stirred at 0 °C for 1/2 hour, then warmed to room temperature and stirred overnight over argon. The reaction was quenched with 100ml of saturated ammonium chloride. The organic layer was washed with 2 x 150ml of brine, then dried over sodium sulfate to yield 42.72g of a red oil. The oil was chromatographed on silica gel using 10% methyl t-butyl ether/90% 1,1,2-trichlorotrifluoroethane as eluant. The product, 1-(5-bromothienyl)-1-(hydroxy)-3-butyne was isolated as a yellow oil and is represented by the formula

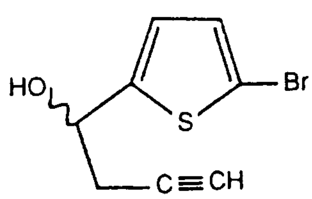

(b) The product of Example 24 (4.63g, 20.03 mmol) in 20ml of dry DMF was added to a 100ml round bottomed flask. The solution was cooled in an ice bath and imidazole (3.00g, 44.06 mmol) was added all at once. After the solid had dissolved, t-butyldimethylchlorosilane (3.32g, 22.03 mmol) was added in one portion. The solution was stirred in the ice bath under argon for 10 min., then stirred at room temperature for 2 hours. TLC in toluene showed no starting material. The reaction mixture was poured into 400ml of

diethyl ether and a yellow gel formed. About 20ml of water was added. The layers were separated, and the organic layer was washed 3X with 75ml of water, 1X with 75ml brine, dried over magnesium sulfate. The solvent was removed under vacuum to give a yellow oil. Chromatography of the yellow oil on a silica gel column packed in 10% ether/hexane gave 6.48g of the product, [[1-(5-bromo-2-thienyl)- 3-butynyl]-oxy](1,1-dimethylethyl)dimethylsilane, as a yellow oil. The product is represented by the formula

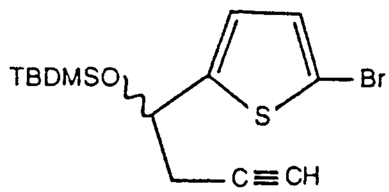

(c) A 1.6M solution of n-butyl lithium in hexane (0.22ml, 0.35 mmole) was added to a solution of diisopropylamine (0.06ml, 0.43 mmol) in THF at 0 ° C. The mixture was stirred for 1/2 hour at 0 ° C, then cooled to -78 ° C. The product from Example 24(b) (0.1g, 0.29 mmol) in 1.0 ml of THF was added over a 1 minute period. The reaction mixture was stirred 5 minutes at -78 ° C, then warmed to -20 ° C and stirred at -20 ° C for 1/2 hour. 1-Iodopentane (0.1ml, 0.75 mmole) was added, followed by 0.5ml of hexamethyl phosphoric triamide (HMPA). The reaction was allowed to warm to room temperature and was stirred at room temperature overnight. The reaction mixture was quenched with water. The reaction mixture was poured into hexane and then extracted 4X with water and 1X with brine, then dried over magnesium sulfate. Removal of the solvent gave the title compound as a red oil.

Example 25

Methyl 7-[5-[1-[[(1,1-dimethylethyl)dimethylsilyl]oxy]-3-nonynyl]-2-thienyl]-5-hydroxy-6-heptynoate

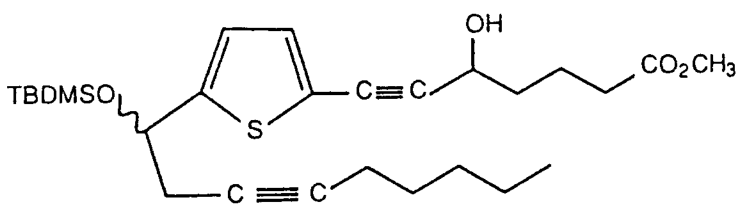

[[1-(5-bromo-2-thienyl)-3-nonyl]oxy](1,1-dimethylethyl)dimethylsilane (145mg, 0.35 mmol), and methyl 5-hydroxy-6-heptynoate (60mg, 0.38 mmol) were dissolved in distilled diisopropylamine (10ml) containing Pd-$(PPh_3)_4$ (25mg). The mixture was heated at 100 ° C in a heavy walled Pyrex® tube for 2 hr. The mixture was cooled and partitioned between ether and water. The organic layer was washed with brine, dried over sodium sulfate, evaporated in vacuo and the residue purified by chromatography on silica gel (ethyl acetate/hexane 2:8). The product was obtained as a yellow oil, 40mgs.

| Microanalysis | | |
|---|---|---|
| Calculated | C 66.07, | H 8.63 |
| Found | C 66.27, | H 8.98 |

Example 26

Methyl 7-[5-[1-[[(1,1-dimethylethyl)dimethylsilyl]oxy]-3-nonenyl]-2-thienyl]-5-hydroxy-6-heptenoate

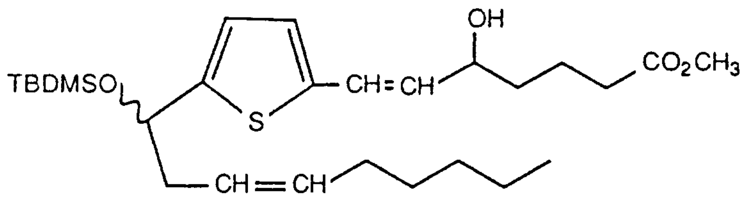

The product of Example 25 (25mg, 0.05 mmol) was dissolved in hexane (2ml), containing Lindlar catalyst (10mg) and quinoline (1 drop). The mixture was evacuated, flushed with hydrogen and then stirred under a hydrogen atmosphere for 24 hr. The reaction mixture was filtered through Celite® filter agent, evaporated in vacuo and the residue purified by chromatography on silica gel (ethyl acetate/hexane 1.5:8.5).

The title compound was obtained as a yellow oil along with 14.4mg of methyl [5-[1-[[(1,1-dimethylethyl)-dimethylsilyl]oxy]-3-nonenyl]-δ-hydroxy-2-thiopheneheptanoate represented by the following formula.

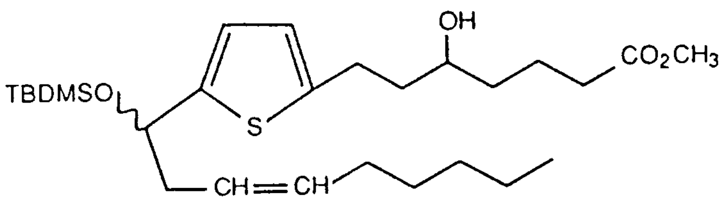

Example 27

5-hydroxy-7-[5-(1-hydroxy-3-nonenyl)-2-thienyl]-6-heptenoic acid

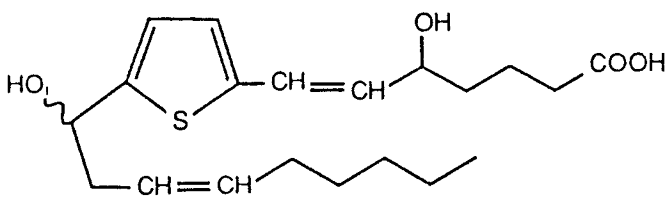

(a) Methyl 7-[5-[1-[[(1,1-dimethylethyl)dimethylsilyl]oxy]-3-nonenyl-2-thienyl]-5-hydroxy-6-heptenoate (20mg) was dissolved in dry THF (1ml) containing 5 equivalents of a 1M solution of tetrabutylammonium fluoride in THF. The mixture was stirred until all starting material had disappeared and then partitioned between ethyl acetate and water. The solvent was dried over sodium sulfate and evaporated to afford a crude residue which was purified by radial band chromatography to afford 2mg of the title compound.

(b) Treatment of the acid prepared in Example 27a with 1 equivalent of an ethereal solution of diazomethane gives the methyl ester.

**Claims**

**Claims for the following Contracting States : AT, BE, CH, DE, FR, GB, IT, LI, NL, SE**

**1.** A compound of the formula:

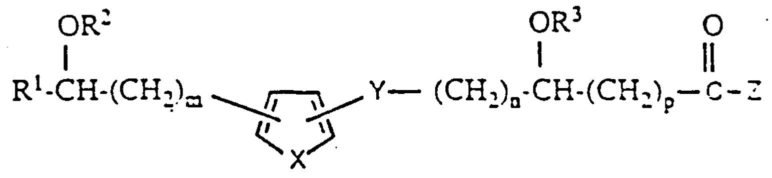

and the pharmaceutically acceptable addition salts thereof;

wherein $R^1$ is lower alkyl having 1-10 carbon atoms; lower alkenyl having 2-10 carbon atoms; lower alkynyl having 2-10 carbon atoms; lower alkadienyl having 3-10 carbon atoms; lower alkadiynyl having 4-10 carbon atoms; or alkenynyl having 4-10 carbon atoms;

wherein $R^2$ and $R^3$ are the same or different and represent hydrogen or lower alkyl having 1-6 carbon atoms;

wherein X is CH = CH, S, or O;

wherein Y is CH = CH or C≡C;

wherein Z is $OR^4$ or $NR^5R^6$, and wherein $R_4$ represents H, lower alkyl having 1-6 carbon atoms, or a pharmaceutically acceptable cation, and wherein $R^5$ and $R_6$ act independently and represent H or lower alkyl having 1-6 carbon atoms, or wherein $R^5$ and $R^6$ may act together with N to form a cycloamine of the formula:

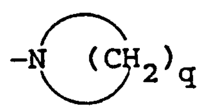

wherein q is an integer from 2-5;

wherein m and n are the same or different and either 1 or 0; and

wherein p is an integer from 1 to 5, except the compound methyl-7-[4-(hydroxynonyl)phenyl]-5-hydroxy-6-heptynoate.

**2.** A compound according to Claim 1 wherein X is CH = CH.

**3.** A compound of the formula:

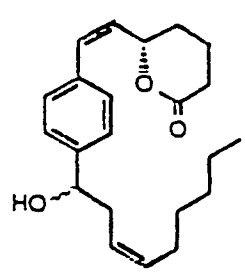

**4.** A compound according to Claim 1 of the formula:

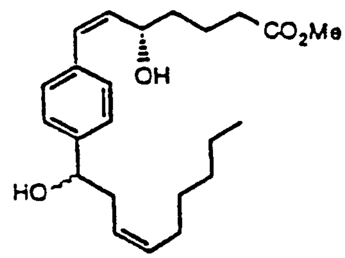

5. A compound according to Claim 1 of the formula:

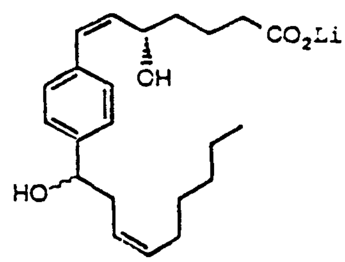

6. A compound according to Claim 1 of the formula:

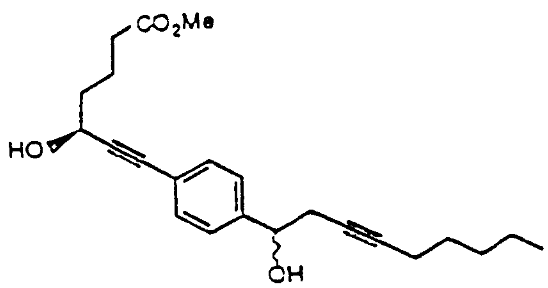

7. A compound according to Claim 1 of the formula:

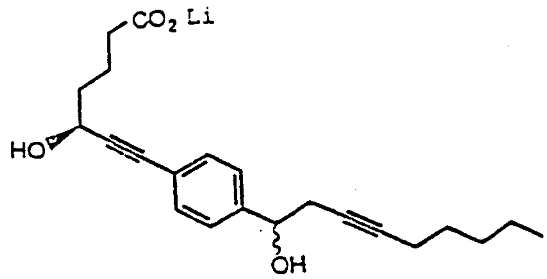

8. A compound according to Claim 1 of the formula:

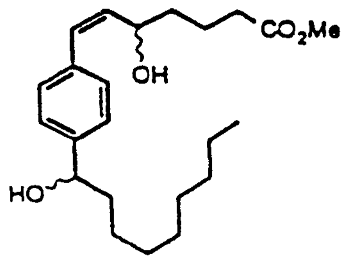

9. A compound according to Claim 1 of the formula:

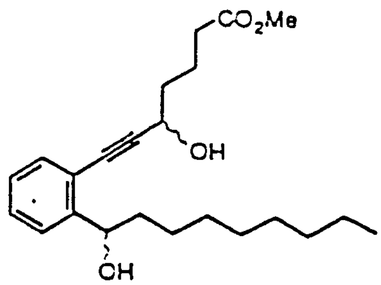

10. A compound according to Claim 1 of the formula:

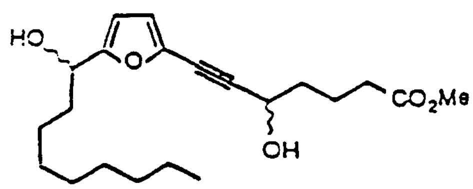

11. A compound according to Claim 1 of the formula:

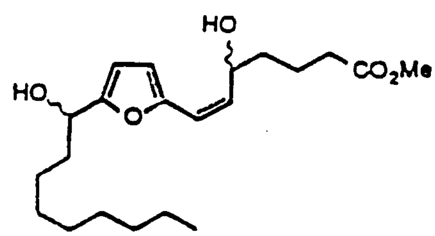

12. A compound according to Claim 1 of the formula:

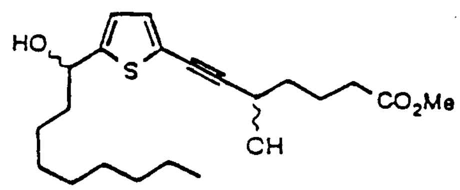

13. A compound according to Claim 1 of the formula:

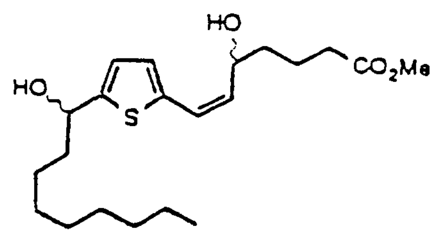

14. A pharmaceutical composition comprising a compound according to Claim 1 and a non-toxic pharmaceutically acceptable carrier.

15. A pharmaceutical composition according to Claim 14 wherein said compound is of the formula:

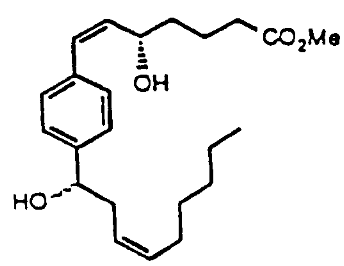

16. A pharmaceutical composition according to Claim 14 wherein said compound is of the formula:

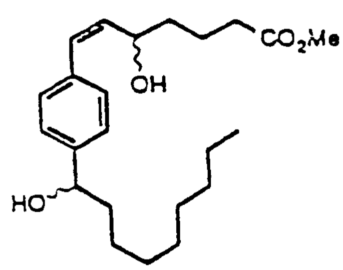

17. A pharmaceutical composition according to Claim 14 wherein said compound is of the formula:

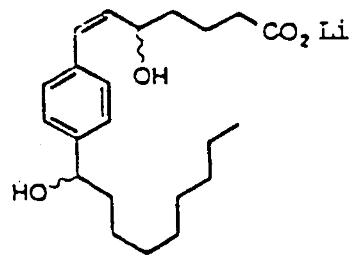

**18.** A pharmaceutical composition according to Claim 14 wherein said compound is of the formula:

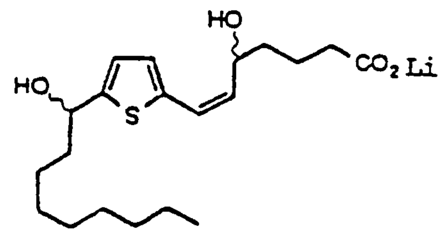

**19.** A pharmaceutical composition, according to Claim 14 which is in oral unit dosage form.

**20.** Use of a compound according to Claim 1 for the production of a medicament for treating an inflammatory condition in mammals.

**21.** Use according to Claim 20 wherein said inflammatory condition is ulcerative colitis.

**22.** Use according to Claim 20 wherein said inflammatory condition is Crohn's disease.

**23.** Use according to Claim 20 wherein said inflammatory condition is psoriasis.

**24.** A compound according to Claim 1 which is 5-hydroxy-7-[5-(1-hydroxy-3-nonenyl)-2-thienyl]-6-heptenoic acid.

**25.** A pharmaceutical composition according to Claim 14 wherein said compound is 5-hydroxy-7-[5-(1-hydroxy-3-nonenyl)-2-thienyl]-6-heptenoic acid.

**26.** Use of methyl-7-[4-(hydroxynonyl)phenyl]-5-hydroxy-6-heptynoate for preparing a medicament for treating an inflammatory condition in mammals.

**Claims for the following Contracting State : ES**

**1.** A process for preparing a compound of the formula:

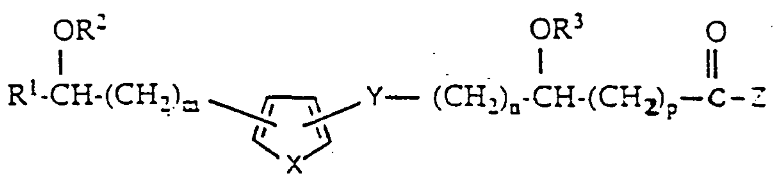

and the pharmaceutically acceptable addition salts thereof;

wherein $R^1$ is lower alkyl having 1-10 carbon atoms; lower alkenyl having 2-10 carbon atoms; lower

alkynyl having 2-10 carbon atoms; lower alkadienyl having 3-10 carbon atoms; lower alkadiynyl having 4-10 carbon atoms; or alkenynyl having 4-10 carbon atoms;

wherein $R^2$ and $R^3$ are the same or different and represent hydrogen or lower alkyl having 1-6 carbon atoms;

wherein X is CH = CH, S, or O;

wherein Y is CH = CH or C≡C;

wherein Z is $OR^4$ or $NR^5R^6$, and wherein $R_4$ represents H, lower alkyl having 1-6 carbon atoms, or a pharmaceutically acceptable cation, and wherein $R^5$ and $R_6$ act independently and represent H or lower alkyl having 1-6 carbon atoms, or wherein $R^5$ and $R^6$ may act together with N to form a cycloamine of the formula:

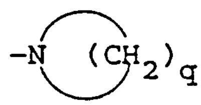

wherein q is an integer from 2-5;

wherein m and n are the same or different and either 1 or 0; and

wherein p is an integer from 1 to 5 with the exception of methyl-7-[4-(hydroxynonyl)phenyl]-5-hydroxy-6-heptynoate, characterized in that a suitably substituted compound of the formula

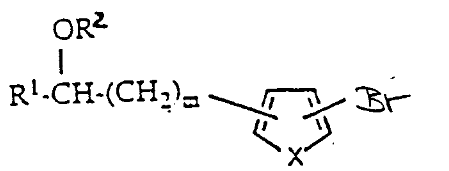

with all the substituents as defined hereinabove
is alkylated in a known manner with a suitably substituted alkyne of the formula

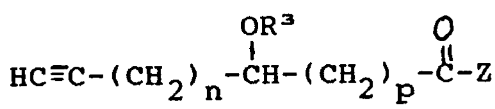

with all the substituents as defined above
whereby the thus obtained compounds subsequently may be hydrated in a known manner to receive compounds wherein Y is CH = CH and/or be transformed in a known manner into their pharmaceutically acceptable addition salts.

**2.** A process according to Claim 1 wherein X of the compound prepared is CH = CH.

**3.** A process according to Claim 1 wherein the compound prepared has the formula

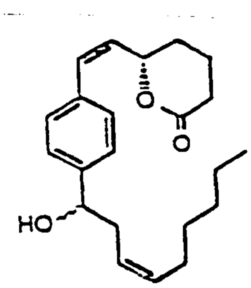

4. A process according to Claim 1 wherein the compound prepared has the formula

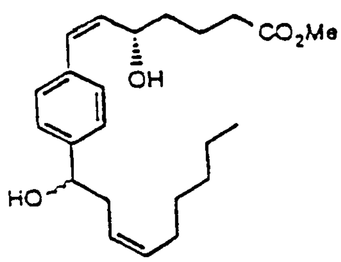

5. A process according to Claim 1 wherein the compound prepared has the formula

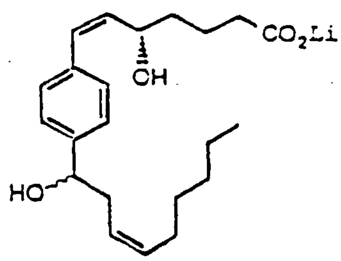

6. A process according to Claim 1 wherein the compound prepared has the formula

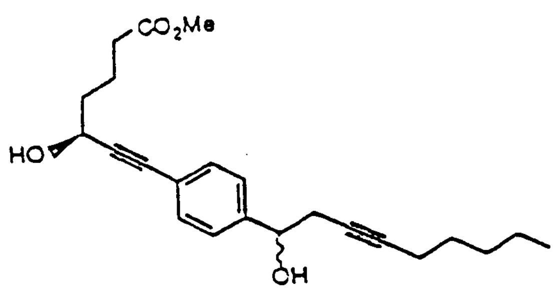

7. A process according to Claim 1 wherein the compound prepared has the formula

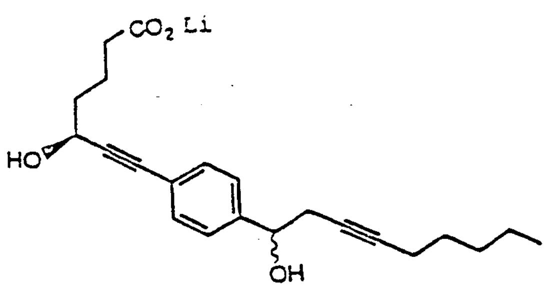

**8.** A process according to Claim 1 wherein the compound prepared has the formula

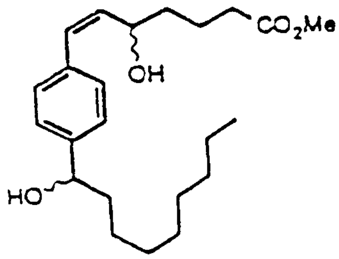

**9.** A process according to Claim 1 wherein the compound prepared has the formula

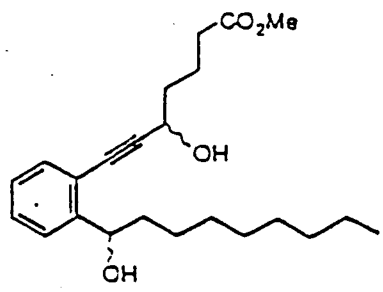

**10.** A process according to Claim 1 wherein the compound prepared has the formula

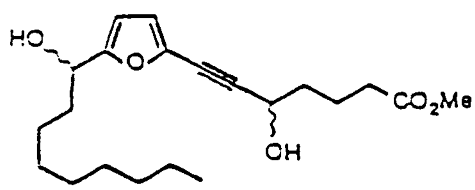

**11.** A process according to Claim 1 wherein the compound prepared has the formula

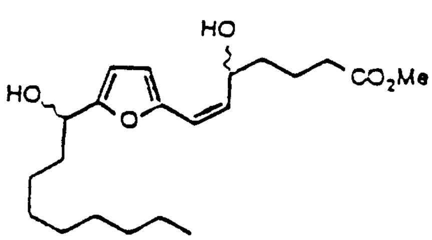

**12.** A process according to Claim 1 wherein the compound prepared has the formula

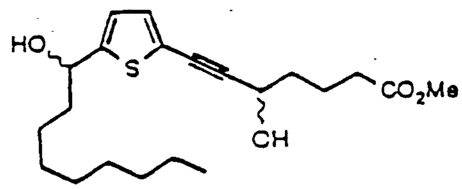

**13.** A process according to Claim 1 wherein the compound prepared has the formula

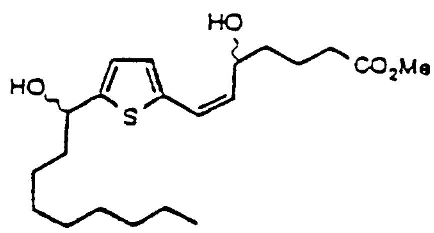

**14.** A process for preparing a pharmaceutical composition comprising admixing a compound prepared according to Claim 1 together with a non-toxic pharmaceutically acceptable carrier.

**15.** A process according to Claim 14 wherein said compound is of the formula:

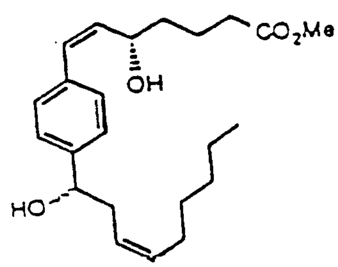

**16.** A process according to Claim 14 wherein said compound is of the formula

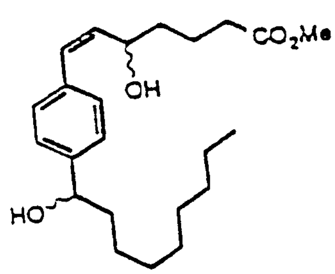

**17.** A process according to Claim 14 wherein said compound is of the formula

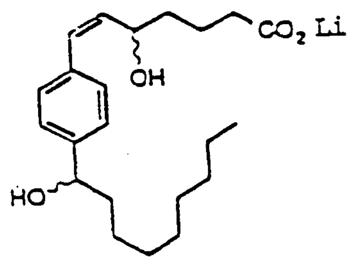

18. A process according to Claim 14 wherein said compound is of the formula

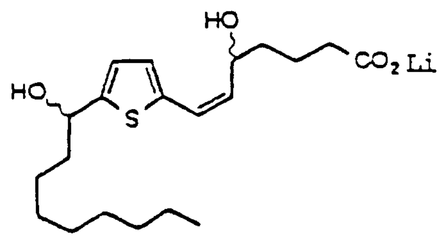

19. A process according to Claim 14 whereby the pharmaceutical composition is in oral unit dosage form.

20. Use of a compound according to Claim 1 for the production of a medicament for treating an inflammatory condition in mammals.

21. Use according to Claim 20 wherein said inflammatory condition is ulcerative colitis.

22. Use according to Claim 20 wherein said inflammatory condition is Crohn's disease.

23. Use according to Claim 20 wherein said inflammatory condition is psoriasis.

24. A process according to Claim 1 wherein the compound prepared is 5-hydroxy-7-[5-(1-hydroxy-3-nonenyl)-2-thienyl]-6-heptenoic acid.

25. A process according to Claim 14 wherein said compound is 5-hydroxy-7-[5-(1-hydroxy-3-nonenyl)-2-thienyl]-6-heptenoic acid.

26. Use of methyl-7-[4-(hydroxynonyl)phenyl]-5-hydroxy-6-heptynoate for preparing a medicament for treating an inflammatory condition in mammals.

**Claims for the following Contracting State : GR**

1. A compound of the formula:

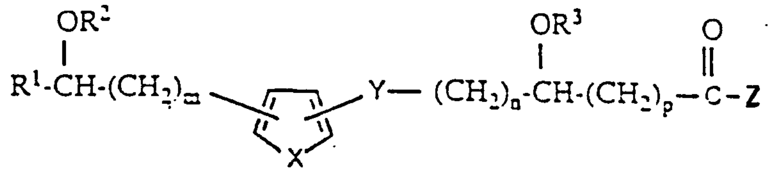

and the pharmaceutically acceptable addition salts thereof;

wherein $R^1$ is lower alkyl having 1-10 carbon atoms; lower alkenyl having 2-10 carbon atoms; lower

alkynyl having 2-10 carbon atoms; lower alkadienyl having 3-10 carbon atoms; lower alkadiynyl having 4-10 carbon atoms; or alkenynyl having 4-10 carbon atoms;

wherein $R^2$ and $R^3$ are the same or different and represent hydrogen or lower alkyl having 1-6 carbon atoms;

wherein X is CH = CH, S, or O;

wherein Y is CH = CH or C≡C;

wherein Z is $OR^4$ or $NR^5R^6$, and wherein $R_4$ represents H, lower alkyl having 1-6 carbon atoms, or a pharmaceutically acceptable cation, and wherein $R^5$ and $R_6$ act independently and represent H or lower alkyl having 1-6 carbon atoms, or wherein $R^5$ and $R^6$ may act together with N to form a cycloamine of the formula:

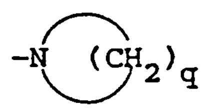

wherein q is an integer from 2-5;

wherein m and n are the same or different and either 1 or 0; and

wherein p is an integer from 1 to 5, except the compound methyl-7-[4-(hydroxynonyl)phenyl]-5-hydroxy-6-heptynoate.

**2.** A compound according to Claim 1 wherein X is CH = CH.

**3.** A compound of the formula:

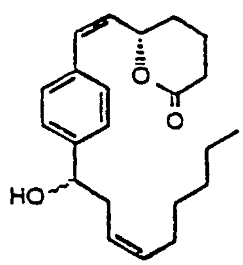

**4.** A compound according to Claim 1 of the formula:

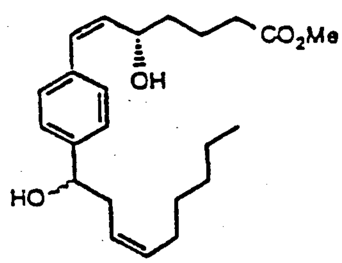

**5.** A compound according to Claim 1 of the formula:

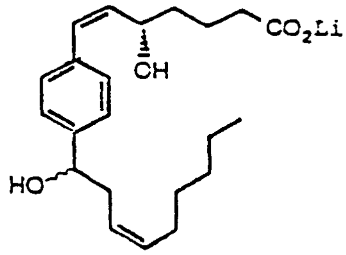

6. A compound according to Claim 1 of the formula:

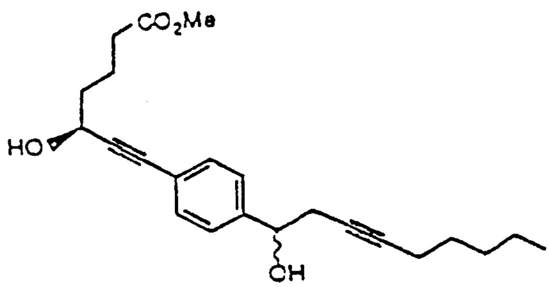

7. A compound according to Claim 1 of the formula:

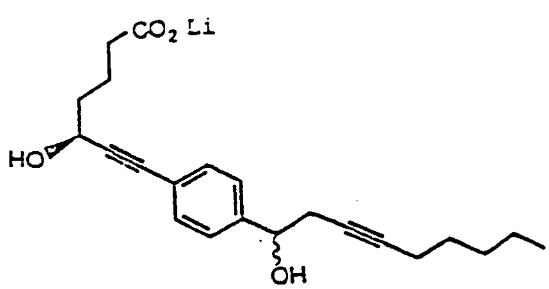

8. A compound according to Claim 1 of the formula:

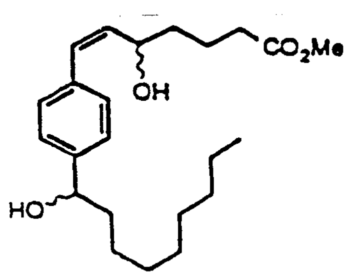

9. A compound according to Claim 1 of the formula:

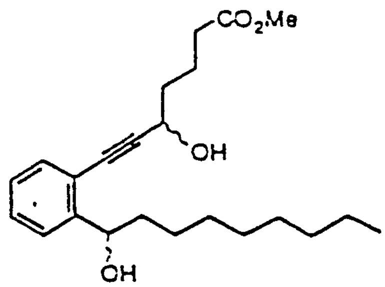

10. A compound according to Claim 1 of the formula:

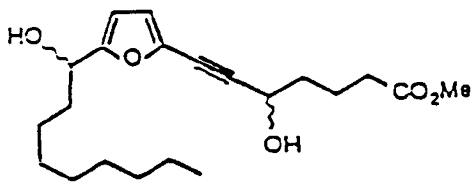

11. A compound according to Claim 1 of the formula:

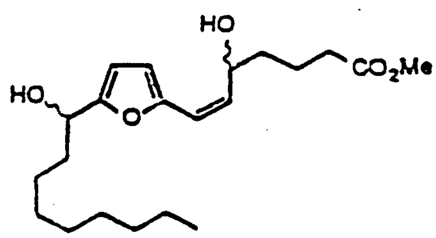

12. A compound according to Claim 1 of the formula:

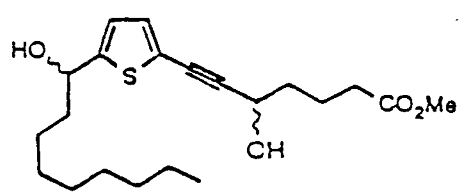

13. A compound according to Claim 1 of the formula:

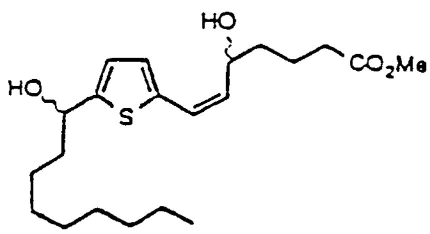

14. A process for preparing a pharmaceutical composition comprising admixing a compound according to Claim 1 together with a non-toxic pharmaceutically acceptable carrier.

15. A process according to Claim 14 wherein said compound is of the formula:

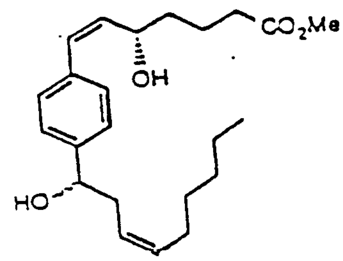

16. A process according to Claim 14 wherein said compound is of the formula

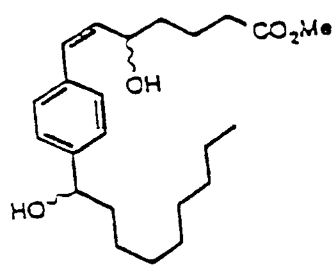

17. A process according to Claim 14 wherein said compound is of the formula

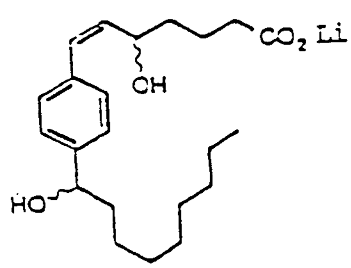

18. A process according to Claim 14 wherein said compound is of the formula

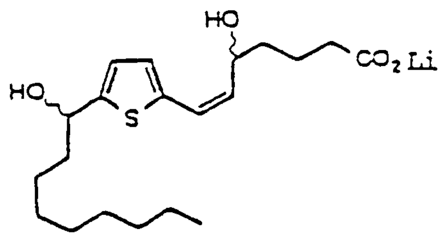

**19.** A process according to Claim 14 whereby the pharmaceutical composition is in oral unit dosage form.

**20.** Use of a compound according to Claim 1 for the production of a medicament for treating an inflammatory condition in mammals.

**21.** Use according to Claim 20 wherein said inflammatory condition is ulcerative colitis.

**22.** Use according to Claim 20 wherein said inflammatory condition is Crohn's disease.

**23.** Use according to Claim 20 wherein said inflammatory condition is psoriasis.

**24.** A compound according to Claim 1 which is 5-hydroxy-7-[5-(1-hydroxy-3-nonenyl)-2-thienyl]-6-heptenoic acid.

**25.** A process according to Claim 14 wherein said compound is 5-hydroxy-7-[5-(1-hydroxy-3-nonenyl)-2-thienyl]-6-heptenoic acid.

**26.** Use of methyl-7-[4-(hydroxynonyl)phenyl]-5-hydroxy-6-heptynoate for preparing a medicament for treating an inflammatory condition in mammals.

**Patentansprüche**
**Patentansprüche für folgende Vertragsstaaten : AT, BE, CH, DE, FR, GB, IT, LI, NL, SE**

**1.** Eine Verbindung der Formel

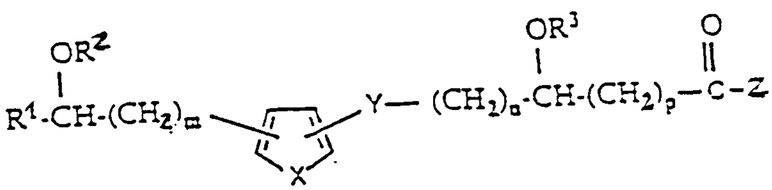

und deren pharmazeutisch verträglichen Additionssalze,
worin $R^1$ eine niedere Alkylgruppe mit 1 bis 10 Kohlenstoffatomen, eine niedere Alkenylgruppe mit 2 bis 10 Kohlenstoffatomen, eine niedere Alkinylgruppe mit 2 bis 10 Kohlenstoffatomen, eine niedere Alkadienylgruppe mit 3 bis 10 Kohlenstoffatomen, eine niedere Alkadiinylgruppe mit 4 bis 10 Kohlenstoffatomen oder eine Alkeninylgruppe mit 4 bis 10 Kohlenstoffatomen bedeutet;
worin $R^2$ und $R^3$ gleich oder verschieden sind und Wasserstoff oder eine niedere Alkylgruppe mit 1 bis 6 Kohlenstoffatomen bedeuten;
worin X CH = CH, S oder O ist;
worin Y CH = CH oder C≡C ist;
worin Z $OR^4$ oder $NR^5R^6$ ist und worin $R^4$ H, eine niedere Alkylgruppe mit 1-6 Kohlenstoffatomen oder ein pharmazeutisch verträgliches Kation darstellt, und worin $R^5$ und $R^6$ unabhängig voneinander H oder eine niedere Alkylgruppe mit 1-6 Kohlenstoffatomen darstellen, oder worin $R^5$ und $R^6$ zusammengenommen mit N ein Cycloamin der Formel

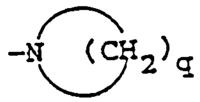

bilden,
worin q eine Zahl von 2-5 ist;
worin m und n gleich oder verschieden sind und entweder 1 oder 0 bedeuten; und
worin p eine Zahl von 1-5 ist, mit Ausnahme der Verbindung 7-[4-(Hydroxynonyl)phenyl]-5-hydroxy-6-heptinsäuremethylester.

**2.** Eine Verbindung gemäß Anspruch 1, worin X CH = CH ist.

**3.** Eine Verbindung der Formel:

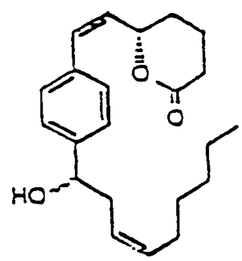

**4.** Eine Verbindung gemäß Anspruch 1 der Formel

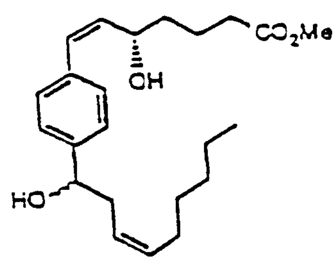

**5.** Eine Verbindung gemäß Anspruch 1 der Formel

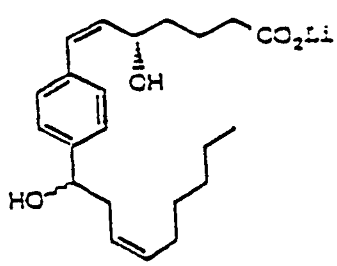

**6.** Eine Verbindung gemäß Anspruch 1 der Formel

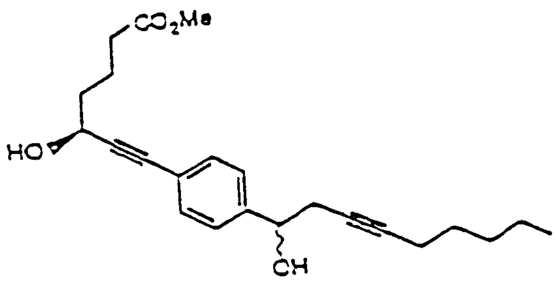

7. Eine Verbindung gemäß Anspruch 1 der Formel

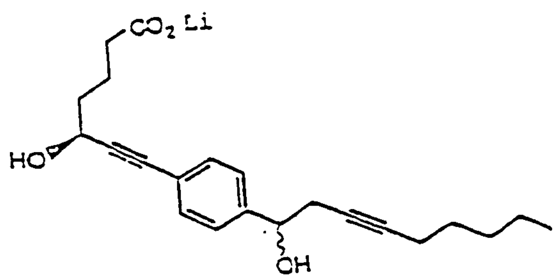

8. Eine Verbindung gemäß Anspruch 1 der Formel

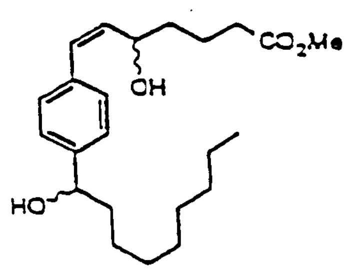

9. Eine Verbindung gemäß Anspruch 1 der Formel

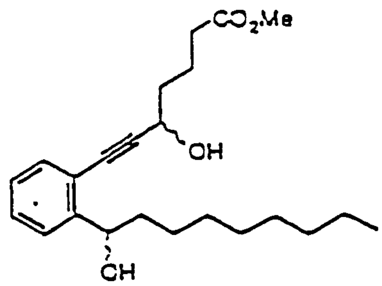

**10.** Eine Verbindung gemäß Anspruch 1 der Formel

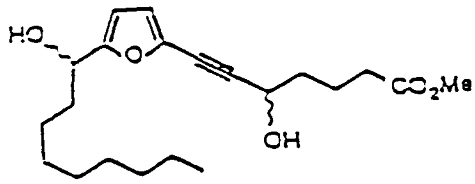

**11.** Eine Verbindung gemäß Anspruch 1 der Formel

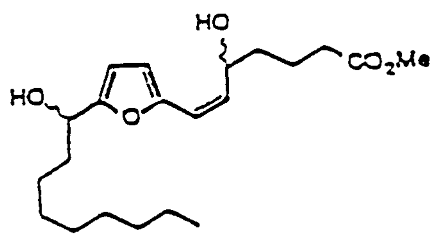

**12.** Eine Verbindung gemäß Anspruch 1 der Formel

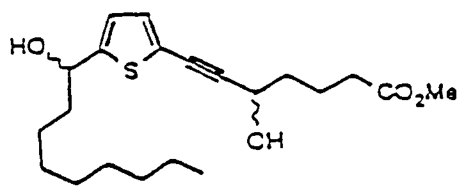

**13.** Eine Verbindung gemäß Anspruch 1 der Formel

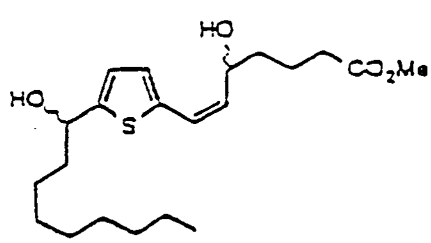

**14.** Eine pharmazeutische Zusammensetzung, umfassend eine Verbindung gemäß Anspruch 1 und einen nicht-toxischen pharmazeutisch-zulässigen Träger.

**15.** Eine pharmazeutische Zusammensetzung gemäß Anspruch 14, worin diese Verbindung die Formel aufweist:

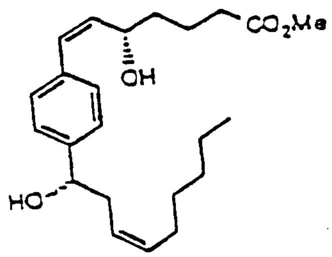

16. Eine pharmazeutische Zusammensetzung gemäß Anspruch 14, worin diese Verbindung die Formel aufweist:

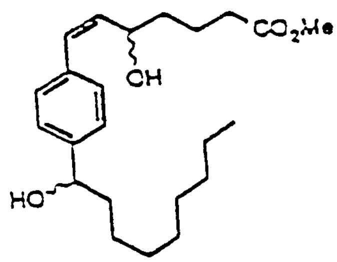

17. Eine pharmazeutische Zusammensetzung gemäß Anspruch 14, worin diese Verbindung die Formel aufweist:

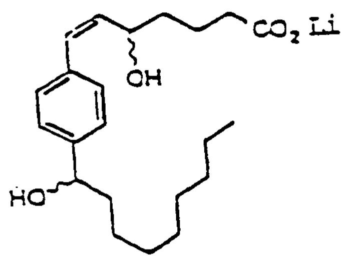

18. Eine pharmazeutische Zusammensetzung gemäß Anspruch 14, worin diese Verbindung die Formel aufweist:

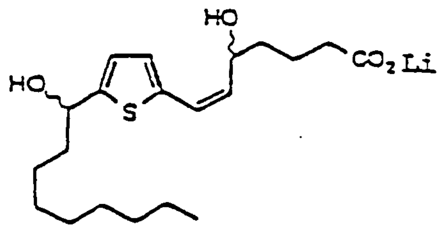

19. Eine pharmazeutische Zusammensetzung gemäß Anspruch 14 in oraler Einheitsdosierungsform.

20. Verwendung einer Verbindung gemäß Anspruch 1 zur Herstellung eines Arzneimittels zur Behandlung

entzündlicher Zustände bei Säugern.

**21.** Verwendung gemäß Anspruch 20, worin dieser entzündliche Zustand eine ulcerative Colitis ist.

**22.** Verwendung gemäß Anspruch 20, worin dieser entzündliche Zustand die Crohn'sche Krankheit ist.

**23.** Verwendung gemäß Anspruch 20, worin dieser entzündliche Zustand Psoriasis ist.

**24.** Eine Verbindung gemäß Anspruch 1, nämlich 5-Hydroxy-7-[5-(1-hydroxy-3-nonenyl)-2-thienyl]-6-heptensäure.

**25.** Eine pharmazeutische Zusammensetzung gemäß Anspruch 14, worin diese Verbindung 5-Hydroxy-7-[5-(1-hydroxy-3-nonenyl)-2-thienyl]-6-heptensäure ist.

**26.** Verwendung von 7-[4-(Hydroxynonyl)phenyl]-5-hydroxy-6-heptinsäuremethylester zur Herstellung eines Arzneimittels zur Behandlung eines entzündlichen Zustandes bei Säugern.

**Patentansprüche für folgenden Vertragsstaat : ES**

**1.** Verfahren zur Herstellung von Verbindungen der Formel

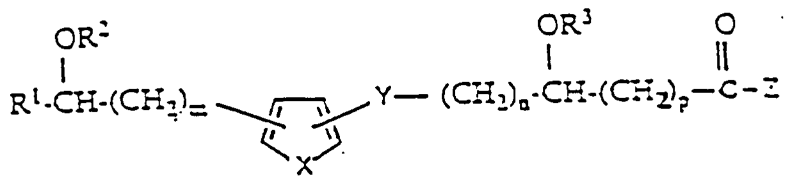

und deren pharmazeutisch verträglichen Additionssalze,
worin $R^1$ eine niedere Alkylgruppe mit 1 bis 10 Kohlenstoffatomen, eine niedere Alkenylgruppe mit 2 bis 10 Kohlenstoffatomen, eine niedere Alkinylgruppe mit 2 bis 10 Kohlenstoffatomen, eine niedere Alkadienylgruppe mit 3 bis 10 Kohlenstoffatomen, eine niedere Alkadiinylgruppe mit 4 bis 10 Kohlenstoffatomen oder eine Alkeninylgruppe mit 4 bis 10 Kohlenstoffatomen bedeutet;
worin $R^2$ und $R^3$ gleich oder verschieden sind und Wasserstoff oder eine niedere Alkylgruppe mit 1 bis 6 Kohlenstoffatomen bedeuten;
worin X CH = CH, S oder O ist;
worin Y CH = CH oder C≡C ist;
worin Z $OR^4$ oder $NR^5R^6$ ist und worin $R^4$ H, eine niedere Alkylgruppe mit 1-6 Kohlenstoffatomen oder ein pharmazeutisch verträgliches Kation darstellt, und worin $R^5$ und $R^6$ unabhängig voneinander H oder eine niedere Alkylgruppe mit 1-6 Kohlenstoffatomen darstellen, oder worin $R^5$ und $R^6$ zusammengenommen mit N ein Cycloamin der Formel

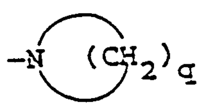

bilden,
worin q eine Zahl von 2-5 ist;
worin m und n gleich oder verschieden sind und entweder 1 oder 0 bedeuten; und
worin p eine Zahl von 1-5 ist, mit Ausnahme der Verbindung 7-[4-(Hydroxynonyl)phenyl]-5-hydroxy-6-heptinsäuremethylester,
dadurch gekennzeichnet, daß eine geeignet substituierte Verbindung der Formel

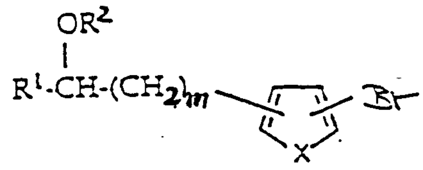

mit allen Substituenten wie oben angegeben,
in an sich bekannter Weise mit einem geeignet substituierten Alkin der Formel

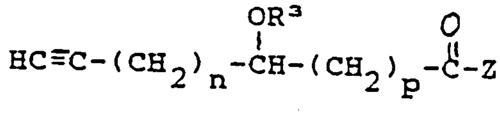

mit allen Substituenten wie oben angegeben, alkyliert wird, wobei die so erhaltenen Verbindungen nachfolgend in an sich bekannter Weise hydriert weden können, wobei Verbindungen mit Y = CH=CH erhalten werden, und/oder in an sich bekannter Weise in deren pharmazeutisch verträglichen Additionssalze umgewandelt werden.

2. Verfahren gemäß Anspruch 1, worin X der hergestellten Verbindung CH=CH ist.

3. Verfahren gemäß Anspruch 1, worin die hergestellte Verbindung die Formel aufweist:

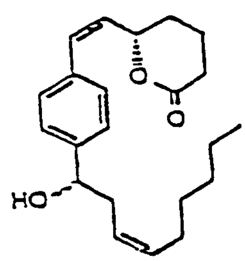

4. Verfahren gemäß Anspruch 1, worin die hergestellte Verbindung die Formel aufweist:

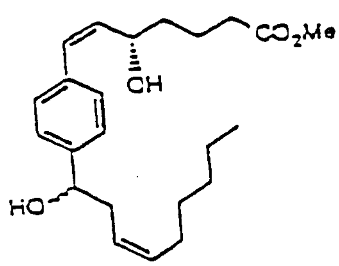

5. Verfahren gemäß Anspruch 1, worin die hergestellte Verbindung die Formel aufweist:

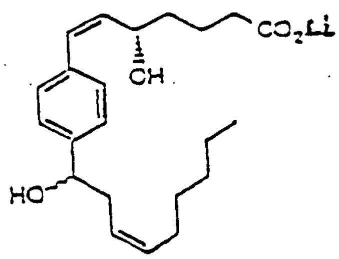

6. Verfahren gemäß Anspruch 1, worin die hergestellte Verbindung die Formel aufweist:

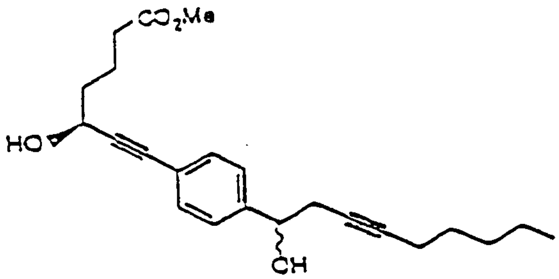

7. Verfahren gemäß Anspruch 1, worin die hergestellte Verbindung die Formel aufweist:

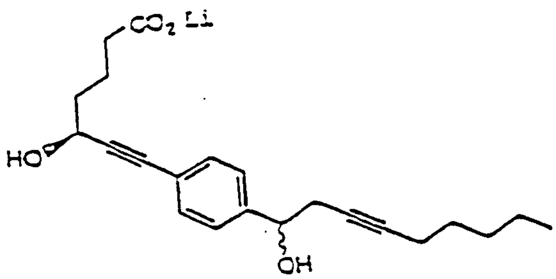

8. Verfahren gemäß Anspruch 1, worin die hergestellte Verbindung die Formel aufweist:

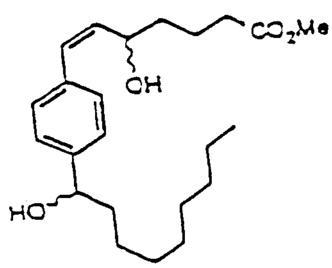

9. Verfahren gemäß Anspruch 1, worin die hergestellte Verbindung die Formel aufweist:

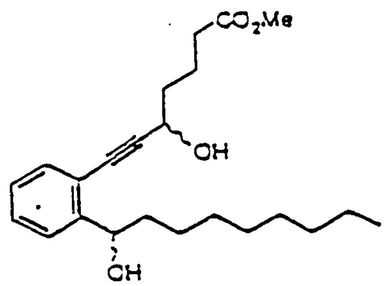

10. Verfahren gemäß Anspruch 1, worin die hergestellte Verbindung die Formel aufweist:

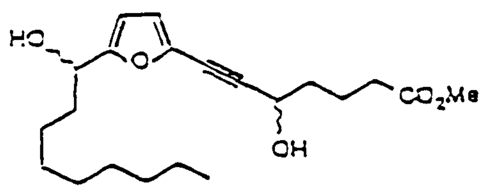

11. Verfahren gemäß Anspruch 1, worin die hergestellte Verbindung die Formel aufweist:

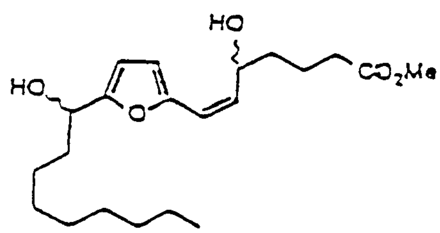

12. Verfahren gemäß Anspruch 1, worin die hergestellte Verbindung die Formel aufweist:

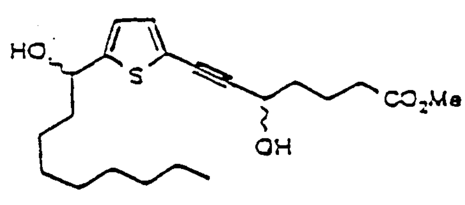

13. Verfahren gemäß Anspruch 1, worin die hergestellte Verbindung die Formel aufweist:

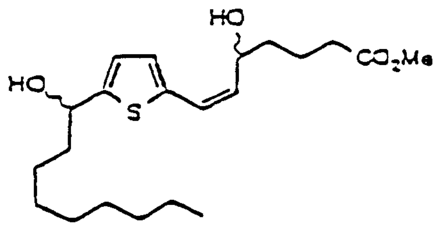

14. Verfahren zur Herstellung einer pharmazeutischen Zusammensetzung, umfassend das Zusammenmischen einer Verbindung gemäß Anspruch 1 mit einem nicht-toxischen pharmazeutisch verträglichen Träger.

15. Verfahren gemäß Anspruch 14, worin diese Verbindung die Formel aufweist:

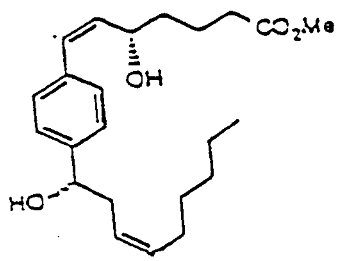

16. Verfahren gemäß Anspruch 14, worin diese Verbindung die Formel aufweist:

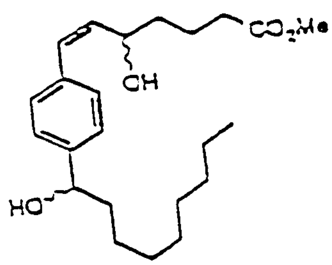

17. Verfahren gemäß Anspruch 14, worin diese Verbindung die Formel aufweist:

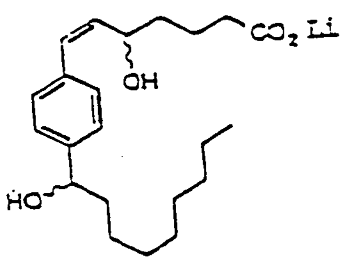

18. Verfahren gemäß Anspruch 14, worin diese Verbindung die Formel aufweist:

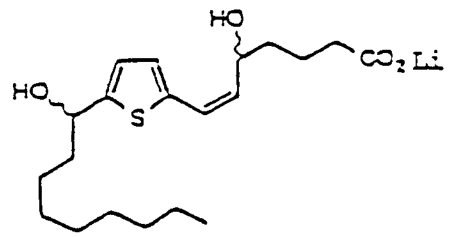

19. Verfahren gemäß Anspruch 14, worin die pharmazeutische Zusammensetzung in oraler Dosierungsform vorliegt.

20. Verwendung einer Verbindung gemäß Anspruch 1 zur Herstellung eines Arzneimittels zur Behandlung entzündlicher Zustände bei Säugern.

21. Verwendung gemäß Anspruch 20, worin dieser entzündliche Zustand eine ulcerative Colitis ist.

22. Verwendung gemäß Anspruch 20, worin dieser entzündliche Zustand die Crohn'sche Krankheit ist.

23. Verwendung gemäß Anspruch 20, worin dieser entzündliche Zustand Psoriasis ist.

24. Verfahren gemäß Anspruch 1, worin die hergestellte Verbindung 5-Hydroxy-7-[5-(1-hydroxy-3-nonenyl)-2-thienyl]-6-heptensäure ist.

25. Verfahren gemäß Anspruch 14, worin die Verbindung 5-Hydroxy-7-[5-(1-hydroxy-3-nonenyl)-2-thienyl]-6-heptensäure ist.

26. Verwendung von 7-[4-(Hydroxynonyl)phenyl]-5-hydroxy-6-heptinsäuremethylester zur Herstellung eines Arzneimittels zur Behandlung eines entzündlichen Zustandes bei Säugern.

**Patentansprüche für folgenden Vertragsstaat : GR**

1. Eine Verbindung der Formel

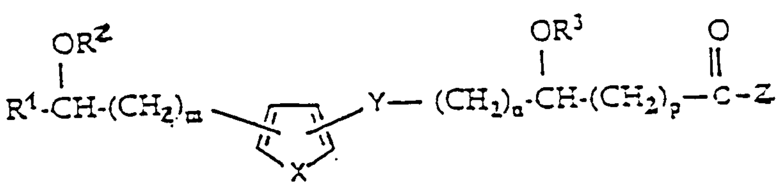

und deren pharmazeutisch verträglichen Additionssalze,
worin $R^1$ eine niedere Alkylgruppe mit 1 bis 10 Kohlenstoffatomen, eine niedere Alkenylgruppe mit 2 bis 10 Kohlenstoffatomen, eine niedere Alkinylgruppe mit 2 bis 10 Kohlenstoffatomen, eine niedere Alkadienylgruppe mit 3 bis 10 Kohlenstoffatomen, eine niedere Alkadiinylgruppe mit 4 bis 10 Kohlenstoffatomen oder eine Alkeninylgruppe mit 4 bis 10 Kohlenstoffatomen bedeutet;
worin $R^2$ und $R^3$ gleich oder verschieden sind und Wasserstoff oder eine niedere Alkylgruppe mit 1 bis 6 Kohlenstoffatomen bedeuten;
worin X CH = CH, S oder O ist;
worin Y CH = CH oder C≡C ist;
worin Z $OR^4$ oder $NR^5R^6$ ist und worin $R^4$ H, eine niedere Alkylgruppe mit 1-6 Kohlenstoffatomen oder ein pharmazeutisch verträgliches Kation darstellt, und worin $R^5$ und $R^6$ unabhängig voneinander H oder eine niedere Alkylgruppe mit 1-6 Kohlenstoffatomen darstellen, oder worin $R^5$ und $R^6$ zusammengenommen mit N ein Cycloamin der Formel

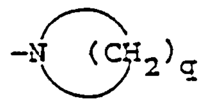

bilden,
worin q eine Zahl von 2-5 ist;
worin m und n gleich oder verschieden sind und entweder 1 oder 0 bedeuten; und
worin p eine Zahl von 1-5 ist, mit Ausnahme der Verbindung 7-[4-(Hydroxynonyl)phenyl]-5-hydroxy-6-heptinsäuremethylester.

**2.** Eine Verbindung gemäß Anspruch 1, worin X CH = CH ist.

**3.** Eine Verbindung der Formel:

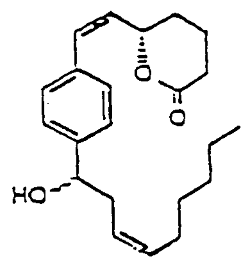

**4.** Eine Verbindung gemäß Anspruch 1 der Formel

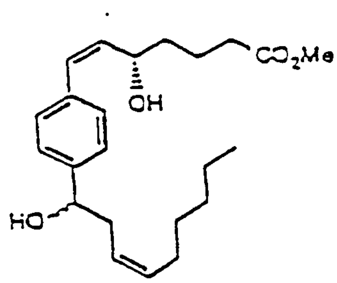

**5.** Eine Verbindung gemäß Anspruch 1 der Formel

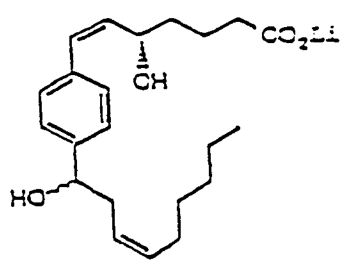

**6.** Eine Verbindung gemäß Anspruch 1 der Formel

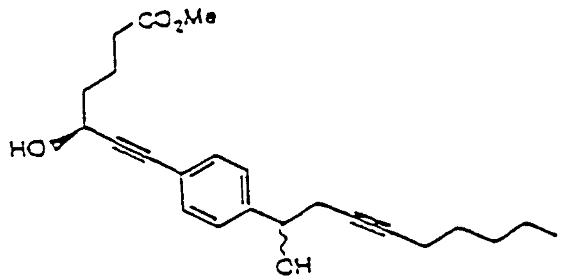

7. Eine Verbindung gemäß Anspruch 1 der Formel

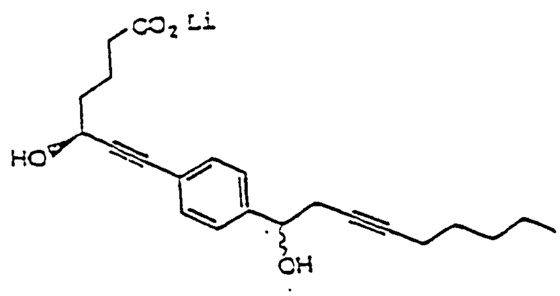

8. Eine Verbindung gemäß Anspruch 1 der Formel

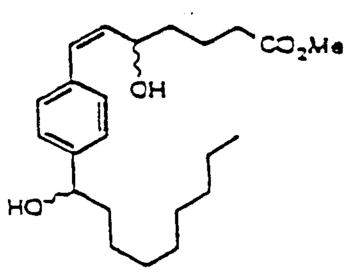

9. Eine Verbindung gemäß Anspruch 1 der Formel

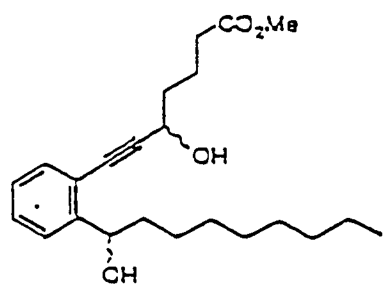

10. Eine Verbindung gemäß Anspruch 1 der Formel

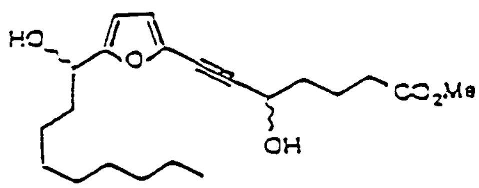

**11.** Eine Verbindung gemäß Anspruch 1 der Formel

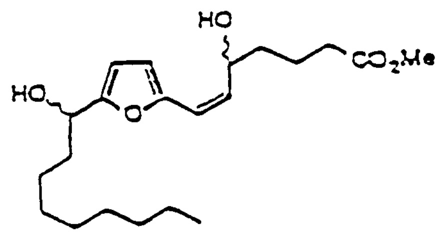

**12.** Eine Verbindung gemäß Anspruch 1 der Formel

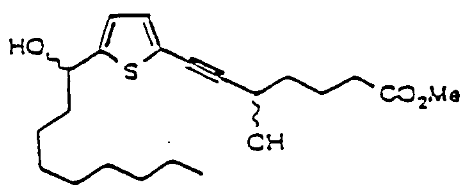

**13.** Eine Verbindung gemäß Anspruch 1 der Formel

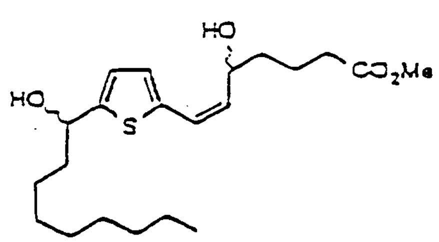

**14.** Verfahren zur Herstellung einer pharmazeutischen Zusammensetzung, umfassend das Zusammenmischen einer Verbindung gemäß Anspruch 1 mit einem nicht-toxischen pharmazeutisch verträglichen Träger.

**15.** Verfahren gemäß Anspruch 14, worin diese Verbindung die Formel aufweist:

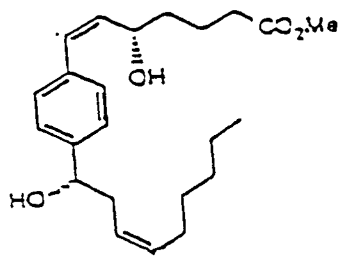

16. Verfahren gemäß Anspruch 14, worin diese Verbindung die Formel aufweist:

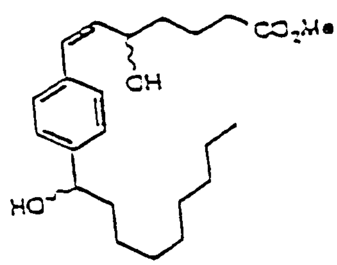

17. Verfahren gemäß Anspruch 14, worin diese Verbindung die Formel aufweist:

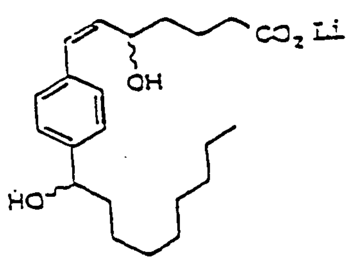

18. Verfahren gemäß Anspruch 14, worin diese Verbindung die Formel aufweist:

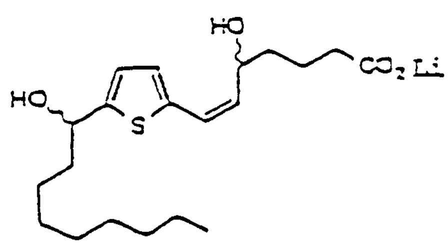

19. Verfahren gemäß Anspruch 14, worin die pharmazeutische Zusammensetzung in oraler Dosierungsform vorliegt.

20. Verwendung einer Verbindung gemäß Anspruch 1 zur Herstellung eines Arzneimittels zur Behandlung entzündlicher Zustände bei Säugern.

**21.** Verwendung gemäß Anspruch 20, worin dieser entzündliche Zustand eine ulcerative Colitis ist.

**22.** Verwendung gemäß Anspruch 20, worin dieser entzündliche Zustand die Crohn'sche Krankheit ist.

**23.** Verwendung gemäß Anspruch 20, worin dieser entzündliche Zustand Psoriasis ist.

**24.** Eine Verbindung gemäß Anspruch 1, nämlich 5-Hydroxy-7-[5-(1-hydroxy-3-nonenyl)-2-thienyl]-6-heptensäure.

**25.** Verfahren gemäß Anspruch 14, worin die Verbindung 5-Hydroxy-7-[5-(1-hydroxy-3-nonenyl)-2-thienyl]-6-heptensäure ist.

**26.** Verwendung von 7-[4-(Hydroxynonyl)phenyl]-5-hydroxy-6-heptinsäuremethylester zur Herstellung eines Arzneimittels zur Behandlung eines entzündlichen Zustandes bei Säugern.

**Revendications**

**Revendications pour les Etats contractants suivants : AT, BE, CH, DE, FR, GB, IT, LI, NL, SE**

**1.** Composé de formule :

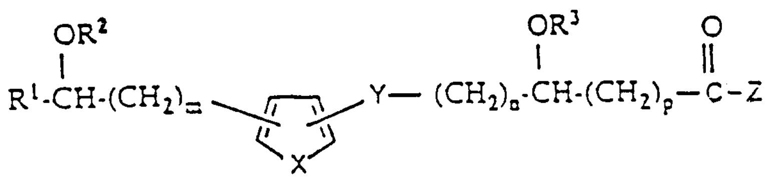

et leurs sels d'addition pharmaceutiquement acceptables ;
(formule dans laquelle) $R^1$ représente un groupe alkyle inférieur ayant 1 à 10 atomes de carbone ; alcényle inférieur ayant 2 à 10 atomes de carbone ; alcynyle inférieur ayant 2 à 10 atomes de carbone ; alcadiényle inférieur ayant 3 à 10 atomes de carbone ; alcadiynyle inférieur ayant 4 à 10 atomes de carbone ou alcénynyle ayant 4 à 10 atomes de carbone ;
$R^2$ et $R^3$, identiques ou différents, représentent chacun un atome d'hydrogène ou un groupe alkyle inférieur ayant 1 à 6 atomes de carbone ;
X représente CH = CH, S ou O ;
Y représente CH =CH ou C≡C ;
Z représente $OR^4$ ou $NR^5R^6$, le symbole $R^4$ représentant H, un groupe alkyle inférieur ayant 1 à 6 atomes de carbone ou un cation pharmaceutiquement acceptable, et les symboles $R^5$ et $R^6$ représentant chacun, indépendamment, un atome de H ou un groupe alkyle inférieur ayant 1 à 6 atomes de carbone, ou bien $R^5$ et $R^6$ peuvent former avec N une cycloamine de formule :

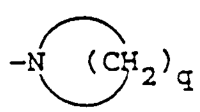

dans laquelle q est un nombre entier valant 2 à 5 ;
m et n sont des nombres égaux ou différents et valant chacun 1 ou 0 ; et
p est un nombre entier valant 1 à 5, à l'exception du composé 7-[4-(hydroxynonyl)phényl]-5-hydroxy-6-heptynoate de méthyle.

**2.** Composé selon la revendication 1, dans lequel X représente CH = CH.

**3.** Composé de formule :

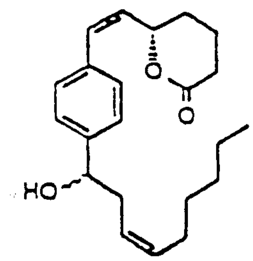

4. Composé selon la revendication 1, répondant à la formule :

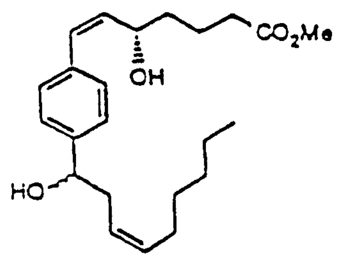

5. Composé selon la revendication 1, répondant à la formule :

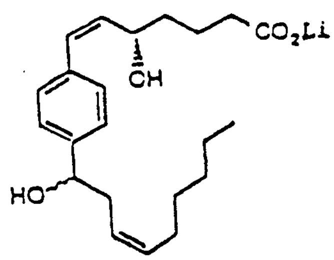

6. Composé selon la revendication 1, répondant à la formule :

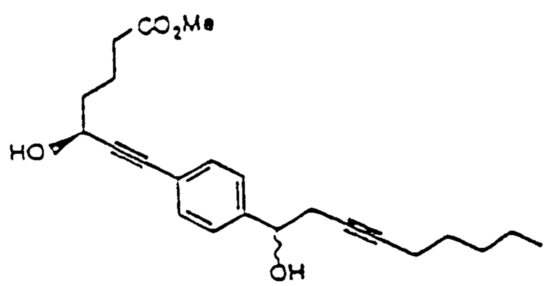

7. Composé selon la revendication 1, répondant à la formule :

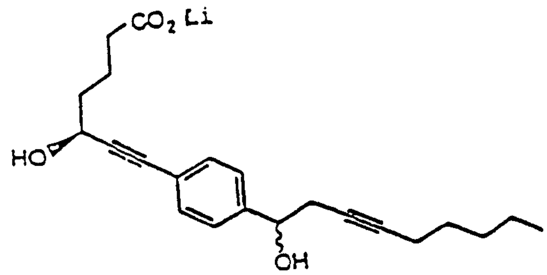

8. Composé selon la revendication 1, répondant à la formule :

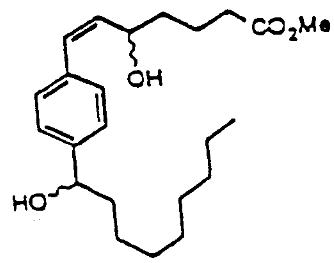

9. Composé selon la revendication 1, répondant à la formule :

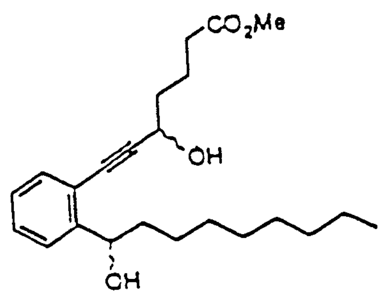

10. Composé selon la revendication 1, répondant à la formule :

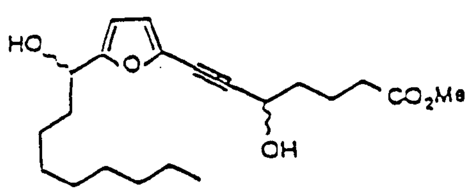

11. Composé selon la revendication 1, répondant à la formule :

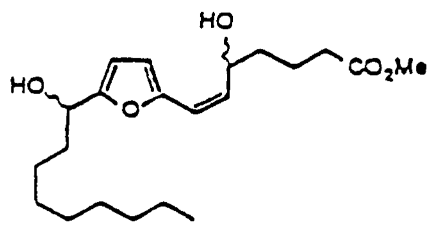

12. Composé selon la revendication 1, répondant à la formule :

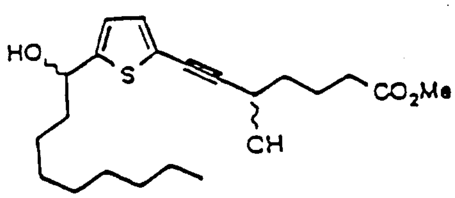

13. Composé selon la revendication 1, répondant à la formule :

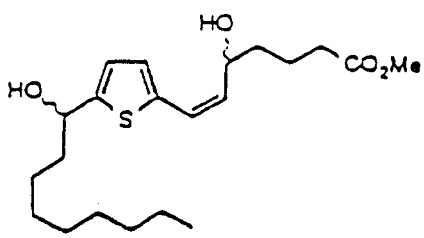

14. Composition pharmaceutique, comprenant un composé selon la revendication 1 et un excipient, véhicule ou support pharmaceutiquement acceptable et non toxique.

15. Composition pharmaceutique selon la revendication 14, dans laquelle ledit composé a pour formule :

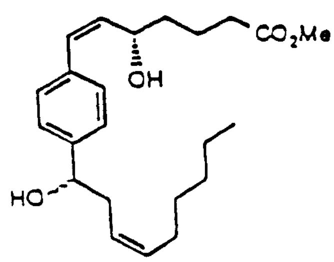

16. Composition pharmaceutique selon la revendication 14, dans laquelle ledit composé a pour formule :

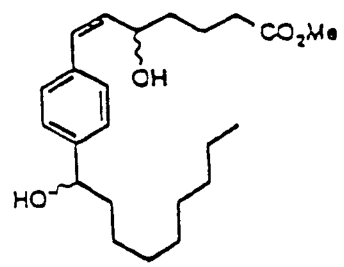

**17.** Composition pharmaceutique selon la revendication 14, dans laquelle ledit composé a pour formule :

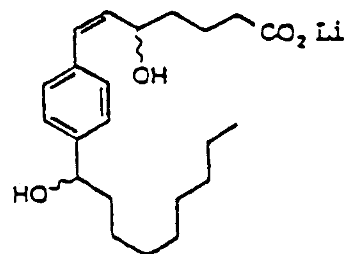

**18.** Composition pharmaceutique selon la revendication 14, dans laquelle ledit composé a pour formule :

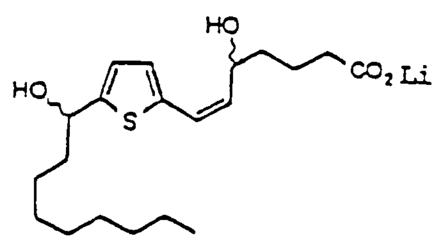

**19.** Composition pharmaceutique selon la revendication 14, qui est sous forme de doses unitaires pour administration par voie orale.

**20.** Utilisation d'un composé selon la revendication 1, pour la production d'un médicament destiné à traiter un état inflammatoire chez les mammifères.

**21.** Utilisation selon la revendication 20, dans laquelle ledit état inflammatoire est de la colite ulcéreuse ou rectocolite hémorragique.

**22.** Utilisation selon la revendication 20, dans laquelle ledit état inflammatoire est la maladie de Crohn.

**23.** Utilisation selon la revendication 20, dans laquelle ledit état inflammatoire est du psoriasis.

**24.** Composé selon la revendication 1, qui est l'acide 5-hydroxy-7-[5-(1-hydroxy-3-nonényl)-2-thiényl]-6-hepténoïque.

**25.** Composition pharmaceutique selon la revendication 14, dans laquelle ledit composé est l'acide 5-hydroxy-7-[5-(1-hydroxy-3-nonényl)-2-thiényl]-6-hepténoïque.

**26.** Utilisation du 7-[4-(hydroxynonyl)phényl]-5-hydroxy-6-heptynoate de méthyle pour préparer un médicament destiné à traiter un état inflammatoire chez des mammifères.

**Revendications pour l'Etat contractant suivant : ES**

**1.** Composé de formule :

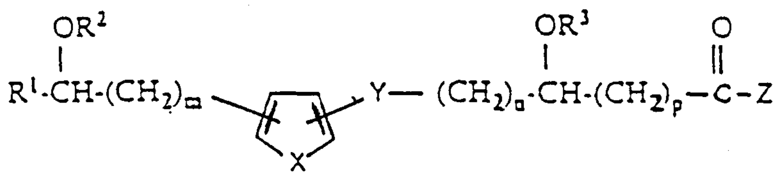

et leurs sels d'addition pharmaceutiquement acceptables ;
(formule dans laquelle) $R^1$ représente un groupe alkyle inférieur ayant 1 à 10 atomes de carbone ; alcényle inférieur ayant 2 à 10 atomes de carbone ; alcynyle inférieur ayant 2 à 10 atomes de carbone ; alcadiényle inférieur ayant 3 à 10 atomes de carbone ; alcadiynyle inférieur ayant 4 à 10 atomes de carbone ou alcénynyle ayant 4 à 10 atomes de carbone ;
$R^2$ et $R^3$, identiques ou différents, représentent chacun un atome d'hydrogène ou un groupe alkyle inférieur ayant 1 à 6 atomes de carbone ;
X représente CH =CH, S ou O ;
Y représente CH=CH ou C≡C ;
Z représente $OR^4$ ou $NR^5R^6$, le symbole $R^4$ représentant H, un groupe alkyle inférieur ayant 1 à 6 atomes de carbone ou un cation pharmaceutiquement acceptable, et les symboles $R^5$ et $R^6$ représentant chacun, indépendamment, un atome de H ou un groupe alkyle inférieur ayant 1 à 6 atomes de carbone, ou bien $R^5$ et $R^6$ peuvent former avec N une cycloamine de formule :

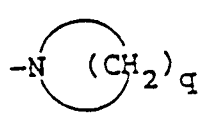

dans laquelle q est un nombre entier valant 2 à 5 ;
m et n sont des nombres égaux ou différents et valant chacun 1 ou 0 ; et
p est un nombre entier valant 1 à 5, à l'exception du composé 7-[4-(hydroxynonyl)phényl]-5-hydroxy-6-heptynoate de méthyle,
procédé caractérisé en ce qu'on soumet un composé de formule :

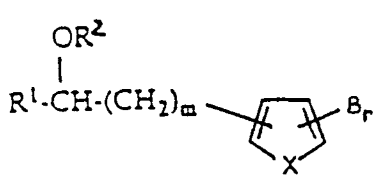

substitué de façon convenable et dont tous les substituants sont tels que définis ci-dessus, à une alkylation effectuée de façon connue à l'aide d'un alcyne substitué de façon appropriée, de formule :

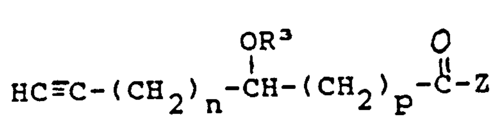

dans laquelle tous les substituants sont tels que définis ci-dessus, et l'on peut ensuite soumettre les composés ainsi obtenus à une hydratation effectuée de manière connue pour obtenir des composés dans lesquels Y représente CH = CH et/ou transformer de façon connue en leurs sels d'addition pharmaceutiquement acceptables.

2. Procédé selon la revendication 1, dans lequel X du composé préparé représente CH = CH.

3. Procédé selon la revendication 1, dans lequel le composé préparé a pour formule :

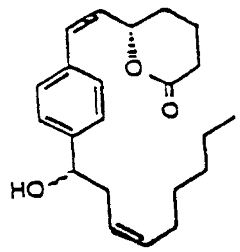

4. Procédé selon la revendication 1, dans lequel le composé préparé a pour formule :

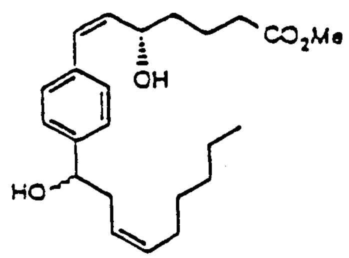

5. Procédé selon la revendication 1, dans lequel le composé préparé a pour formule :

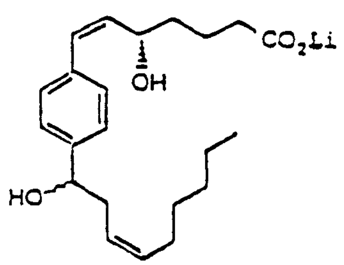

6. Procédé selon la revendication 1, dans lequel le composé préparé a pour formule :

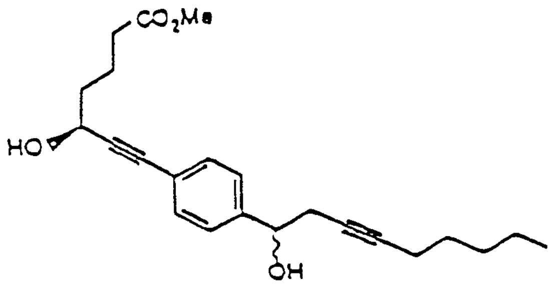

7. Procédé selon la revendication 1, dans lequel le composé préparé a pour formule :

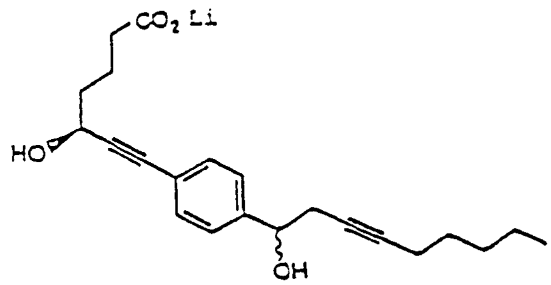

8. Procédé selon la revendication 1, dans lequel le composé préparé a pour formule :

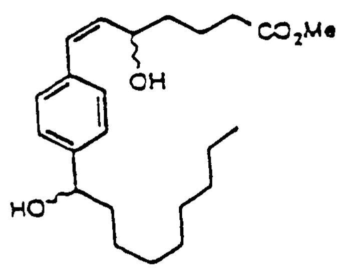

9. Procédé selon la revendication 1, dans lequel le composé préparé a pour formule :

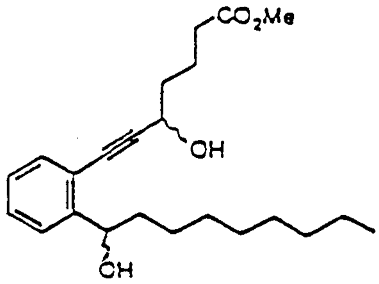

10. Procédé selon la revendication 1, dans lequel le composé préparé a pour formule :

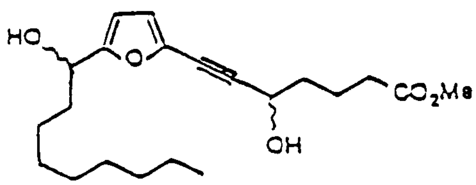

11. Procédé selon la revendication 1, dans lequel le composé préparé a pour formule :

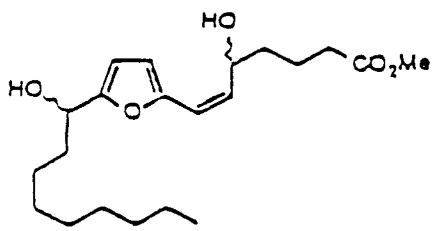

12. Procédé selon la revendication 1, dans lequel le composé préparé a pour formule :

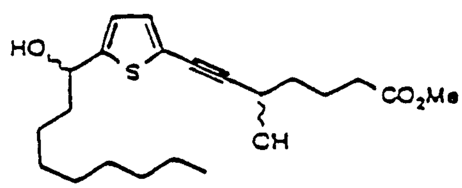

13. Procédé selon la revendication 1, dans lequel le composé préparé a pour formule :

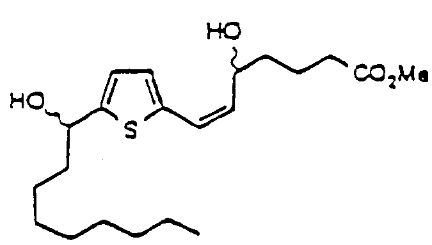

14. Procédé pour préparer une composition pharmaceutique, comprenant le mélangeage d'un composé préparé selon la revendication 1 avec un excipient, support ou véhicule pharmaceutiquement acceptable et non toxique.

15. Procédé selon la revendication 14, dans lequel ledit composé a pour formule :

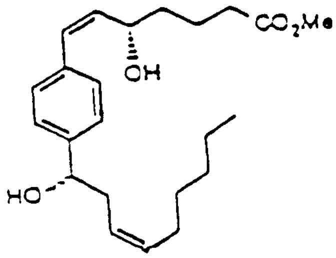

16. Procédé selon la revendication 14, dans lequel ledit composé a pour formule :

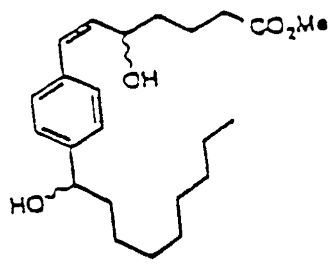

17. Procédé selon la revendication 14, dans lequel ledit composé a pour formule :

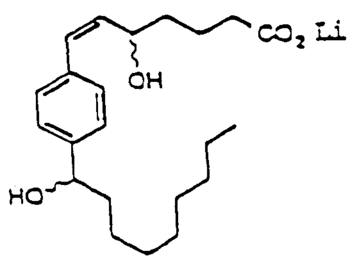

18. Procédé selon la revendication 14, dans lequel ledit composé a pour formule :

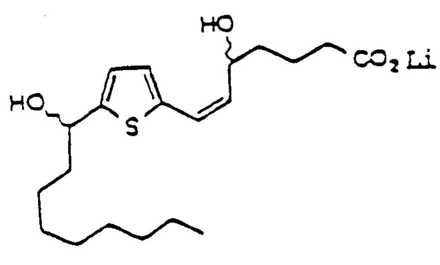

19. Procédé selon la revendication 14, dans lequel la composition pharmaceutique est sous forme de doses unitaires pour administration par voie orale.

20. Utilisation d'un composé (obtenu) selon la revendication 1 pour la production d'un médicament destiné à traiter un état inflammatoire chez les mammifères.

21. Utilisation selon la revendication 20, dans laquelle ledit état inflammatoire est de la colite ulcéreuse ou

de la rectocolite hémorragique.

**22.** Utilisation selon la revendication 20, dans laquelle ledit état inflammatoire est la maladie de Crohn.

**23.** Utilisation selon la revendication 20, dans laquelle ledit état inflammatoire est du psoriasis.

**24.** Procédé selon la revendication 1, dans lequel le composé préparé est l'acide 5-hydroxy-7-[5-(1-hydroxy-3-nonényl)-2-thiényl]-6-hepténoïque.

**25.** Procédé selon la revendication 14, dans lequel ledit composé est l'acide 5-hydroxy-7-(5-(1-hydroxy-3-nonényl)-2-thiényl]-6-hepténoïque.

**26.** Utilisation du 7-[4-(hydroxynonyl)phényl]-5-hydroxy-6-heptynoate de méthyle pour préparer un médicament destiné à traiter un état inflammatoire chez des mammifères.

**Revendications pour l'Etat contractant suivant : GR**

**1.** Composé de formule :

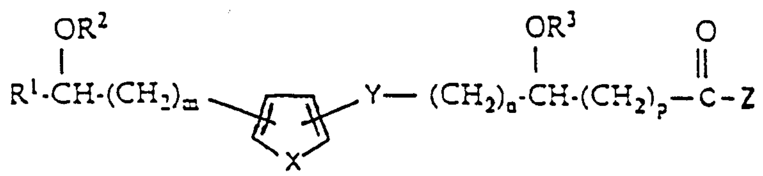

et leurs sels d'addition pharmaceutiquement acceptables ;
(formule dans laquelle) $R^1$ représente un groupe alkyle inférieur ayant 1 à 10 atomes de carbone ; alcényle inférieur ayant 2 à 10 atomes de carbone ; alcynyle inférieur ayant 2 à 10 atomes de carbone ; alcadiényle inférieur ayant 3 à 10 atomes de carbone ; alcadiynyle inférieur ayant 4 à 10 atomes de carbone ou alcénynyle ayant 4 à 10 atomes de carbone ;
$R^2$ et $R^3$, identiques ou différents, représentent chacun un atome d'hydrogène ou un groupe alkyle inférieur ayant 1 à 6 atomes de carbone ;
X représente CH = CH, S ou O ;
Y représente CH = CH ou C≡C ;
Z représente $OR^4$ ou $NR^5R^6$, le symbole $R^4$ représentant H, un groupe alkyle inférieur ayant 1 à 6 atomes de carbone ou un cation pharmaceutiquement acceptable, et les symboles $R^5$ et $R^6$ représentant chacun, indépendamment, un atome de H ou un groupe alkyle inférieur ayant 1 à 6 atomes de carbone, ou bien $R^5$ et $R^6$ peuvent former avec N une cycloamine de formule :

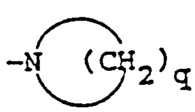

dans laquelle q est un nombre entier valant 2 à 5 ;
m et n sont des nombres égaux ou différents et valant chacun 1 ou 0 ; et
p est un nombre entier valant 1 à 5, à l'exception du composé 7-[4-(hydroxynonyl)phényl]-5-hydroxy-6-heptynoate de méthyle.

**2.** Composé selon la revendication 1, dans lequel X représente CH = CH.

**3.** Composé de formule :

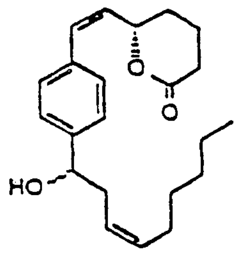

4. Composé selon la revendication 1, répondant à la formule :

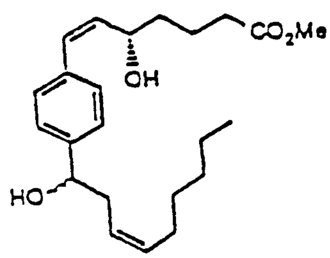

5. Composé selon la revendication 1, répondant à la formule :

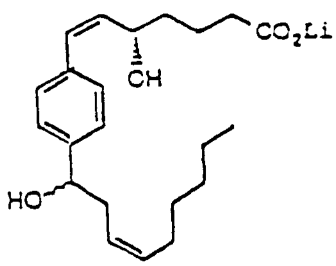

6. Composé selon la revendication 1, répondant à la formule :

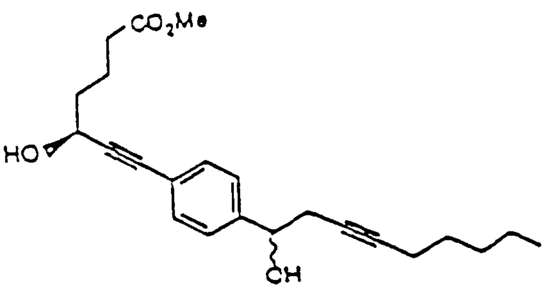

7. Composé selon la revendication 1, répondant à la formule :

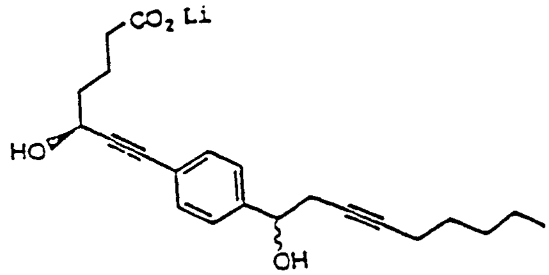

8. Composé selon la revendication 1, répondant à la formule :

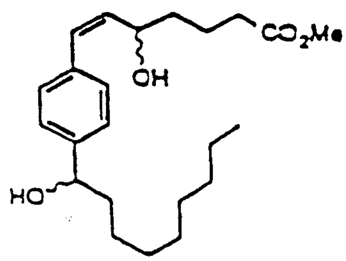

9. Composé selon la revendication 1, répondant à la formule :

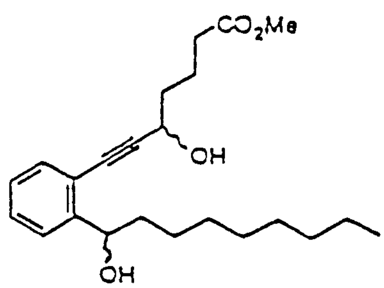

10. Composé selon la revendication 1, répondant à la formule :

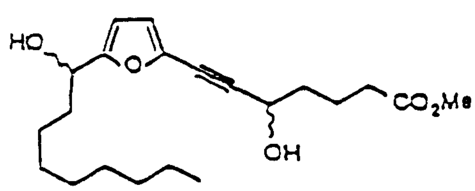

11. Composé selon la revendication 1, répondant à la formule :

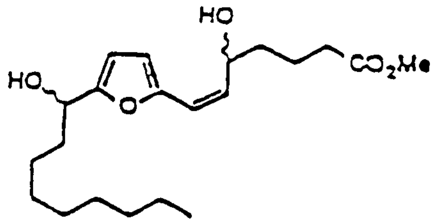

**12.** Composé selon la revendication 1, répondant à la formule :

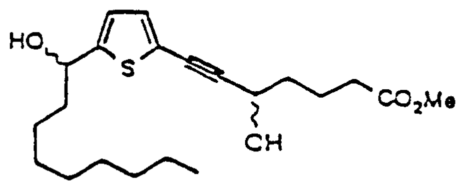

**13.** Composé selon la revendication 1, répondant à la formule :

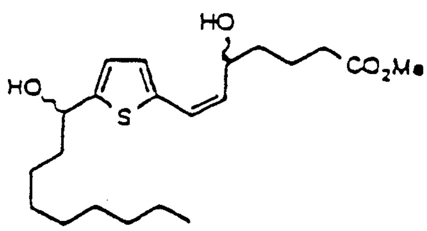

**14.** Procédé pour préparer une composition pharmaceutique, comprenant le mélangeage d'un composé selon la revendication 1 avec un excipient, véhicule ou support non toxique et pharmaceutiquement acceptable.

**15.** Procédé selon la revendication 14, dans lequel ledit composé a pour formule :

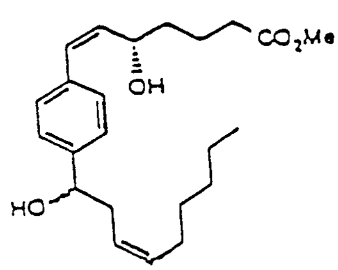

**16.** Procédé selon la revendication 14, dans lequel ledit composé a pour formule :

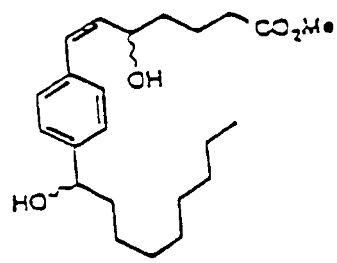

**17.** Procédé selon la revendication 14, dans lequel ledit composé a pour formule :

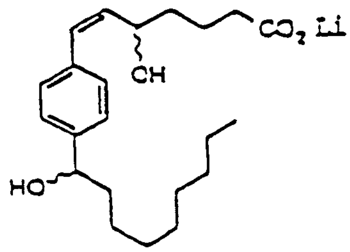

**18.** Procédé selon la revendication 14, dans lequel ledit composé a pour formule :

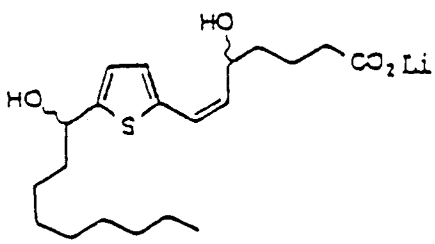

**19.** Procédé selon la revendication 14, dans lequel la composition pharmaceutique est sous forme de doses unitaires pour administration par voie orale.

**20.** Utilisation d'un composé selon la revendication 1, pour la production d'un médicament destiné à traiter un état inflammatoire chez des mammifères.

**21.** Utilisation selon la revendication 20, dans laquelle ledit état inflammatoire est de la colite ulcéreuse ou de la rectocolite hémorragique.

**22.** Utilisation selon la revendication 20, dans laquelle ledit état inflammatoire est la maladie de Crohn.

**23.** Utilisation selon la revendication 20, dans laquelle ledit état inflammatoire est du psoriasis.

**24.** Composé selon la revendication 1, qui est l'acide 5-hydroxy-7-[5-(1-hydroxy-3-nonényl)-2-thiényl]-6-hepténoïque.

**25.** Procédé selon la revendication 14, dans lequel ledit composé est l'acide 5-hydroxy-7-[5-(1-hydroxy-3-nonényl)-2-thiényl]-6-hepténoïque.

**26.** Utilisation du 7-[4-(hydroxynonyl)phényl]-5-hydroxy-6-heptynoate de méthyle pour préparer un médicament destiné à traiter un état inflammatoire chez des mammifères.